# EUROPEAN PATENT SPECIFICATION

(11) **EP 3 413 835 B1**
(45) Date of publication and mention of the grant of the patent: **14.05.2025**
(21) Application number: 17750623.5
(22) Date of filing: 07.02.2017
(51) Int. Cl.: A61C 17/22, A46B 15/00, A61N 5/06

(54) **SHORT WAVELENGTH VISIBLE LIGHT-EMITTING TOOTHBRUSH WITH AN ELECTRONIC SIGNAL INTERLOCK CONTROL**
SICHTBARES LICHT MIT KURZER WELLENLÄNGE EMITTIERENDE ZAHNBÜRSTE MIT VERRIEGELUNGSSTEUERUNG DURCH EIN ELEKTRONISCHES SIGNAL
BROSSE À DENTS ÉMETTRICE DE LUMIÈRE VISIBLE À LONGUEURS D'ONDES COURTES COMPRENANT UNE COMMANDE DE VERROUILLAGE À SIGNAL ÉLECTRONIQUE

(30) Priority: 08.02.2016 US 201615018196; 04.05.2016 US 201662331665 P; 16.12.2016 US 201662435168 P
(43) Date of publication of application: 19.12.2018
(73) Proprietor: Oralucent, Inc., Long Beach CA 90803 (US)
(72) Inventor: BARNES, Mike, Dunellon, FL 34432 (US); LAWRENCE, Timothy, Long Beach, CA 90803 (US); SHEPHERD, Greg, Fruitland Park, FL 34731 (US); ZASTAWNY, Mathieu, Julien, Jean, Emile, Edgewater NJ 07020 (US); DOOLEY, Thomas, Jason, Hoboken, NJ 07030 (US)
(74) Representative: Ashton, Gareth Mark
(86) International application number: PCT/US2017/016805
(87) International publication number: WO 2017/139256

(56) References cited:
- EP-A1- 2 550 935
- WO-A1-2006/098719
- WO-A1-2007/093860
- WO-A1-2015/084962
- US-A1- 2006 281 042
- US-A1- 2008 109 973
- US-A1- 2008 131 834
- US-A1- 2008 276 393
- US-A1- 2011 149 591
- US-A1- 2013 080 295
- US-A1- 2014 323 836
- US-A1- 2015 157 119
- US-A1- 2015 328 803
- US-A1- 2015 351 883

## Description

### Background of the Invention

The present invention relates to increasing the safety of a dental hygiene implement such as a light-emitting manual or an electrically operated motorized toothbrush which emits radiation in the violet and/or blue region of the visible spectrum, between 400nm and 500nm (referred to herein as "visible therapeutic light"), in order to:
- oxidize and destroy potentially harmful bacteria and/or other contaminants or compounds contained within the mouth without harming or destroying human cells;
- exert a phototoxic effect on pathogenic periodontal and oral bacteria such as; P. Gingivalis and F. Nucleatum, and S. Mutans;
- activate a photo catalyst that may be deposited on the teeth and the gums of the person utilizing the toothbrush during normal brushing; and/or
- accelerate the whitening effects of a tooth bleaching agent added to toothpaste or toothgel such as carbamide peroxide or hydrogen peroxide.

The electronic interlock control mechanism in this toothbrush will reduce the possibility of accidental direct eye exposure to high flux visible light radiation emitted from this toothbrush when it is removed from the mouth.

Light-emitting toothbrushes have been developed over the past several years for teeth whitening applications in addition to the known oral hygiene benefits of regular brushing. When combined with a teeth whitening agent such as carbamide peroxide or hydrogen peroxide, studies have shown that light in the 400-500nm range accelerates the whitening effect of these agents. Wolfgang Buchallaa, Thomas Attina: External bleaching therapy with activation by heat, light or laser-A systematic review*;* Karen Luk, D.D.S.; Laura Tam, D.D.S., M.Sc.; Manfred Hubert, Ph.D.: Effect of light energy on peroxide tooth bleaching*.*

In addition, violet light in the 400nm-420nm range has been shown to have a phototoxic effect on pathogenic oral bacteria such as P. Gingivalis, S. Mutans and others. Michelle Maclean, Scott J. MacGregor, John G. Anderson, and Gerry Woolsey: Inactivation of Bacterial Pathogens following Exposure to Light from a 405-Nanometer Light-Emitting Diode Array*.* Doron Steinberg, Daniel Moreinos, John Featherstone, Moshe Shemesh, and Osnat Feuerstein: Genetic and Physiological Effects of Noncoherent Visible Light Combined with Hydrogen Peroxide on Streptococcus mutants in Biofilm*.* The inventors have previously shown the use of a light-emitting diode (LED) within a toothbrush provides anti-microbial properties of benefit to the oral hygiene of the end-user.

Other studies have shown that red and infrared light in the range of 600 nm - 1000 nm ("infrared therapeutic light") provides various oral health benefits and can be useful for treating sensitive teeth, tooth and bone damage, reduction of tooth decay causing bacteria, oral thrush/candidiasis, gum inflammation and oral wounds in soft tissue, ulcers, cold sores, tonsillitis, and other viral/bacterial infections. However, the application of light in this range is generally performed in a controlled laboratory environment or under the supervision of an oral care professional and involves the use of expensive equipment requiring specialized handling to avoid over-exposure, burns or eye-damage.

Current light-emitting toothbrushes have a manual on/off switch which activates the light-emitting device. This manual activation mechanism may lead to a safety risk because the user may activate the light and expose his or her eyes to high levels of light that may be harmful to the retina or optic nerve. The potentially harmful properties of visible light and maximum exposure levels are documented in ANSI standards. François C. Delori, Robert H. Webb, David H. Sliney: Maximum permissible exposures for ocular safety (ANSI 2000), with emphasis on ophthalmic devices*.* David H. Sliney, M.S.: Biohazards of Ultraviolet, Visible and Infrared Radiation*.* For example, the maximum permissible radiant power (thermal and photo-acoustic) entering a dilated pupil is 1.5 x 10⁻⁴ Watts. This limit would be exceeded if a user were to stare at a 420nm LED of 250mW radiant flux at a distance of 10cm for a period of 0.5 seconds. To prevent accidental eye exposure, a special electronic interlock control mechanism has been implemented to keep the optical source turned off if the toothbrush is not inserted in the user's mouth. The control mechanism will turn the optical source off immediately if the toothbrush is removed from the mouth prior to completing the brushing cycle.

A toothbrush is typically used in close proximity to the eyes of the user, and if ocular exposure to the light lasts several seconds, eye damage may occur. Furthermore, the ocular safety risk of manually activated light precludes the use of more powerful light-emitting devices such as high-powered LEDs, laser diodes, or vertical cavity surface emitting lasers, which would increase the teeth-whitening and antimicrobial benefits in proportion to the energy delivered. For example, studies show that effective whitening treatments require a minimum energy density of 30-50 J cm⁻² to produce noticeable shade whitening. However, such energy levels would not be readily achievable with a typical two minute brushing interval using a low-powered LED that would also be safe when directly placed in front of the eyes, even when used over a period of several weeks. Similar limitations exist for the anti-microbial properties of violet light as well.

It is therefore desirable to control the "on" state of the light-emitting device to a time period when it is in use in the oral cavity but to shut "off" this high power light source immediately, when it is removed from the mouth to prevent direct eye exposure. This feature would also extend battery life of a battery operated brush since power is only used to illuminate the light source when in direct contact with the oral cavity.

Ionic toothbrushes operate on the principal of electrostatic attraction. The theory holds that positively charged bacteria as well as acidic compounds with an H+ ion adhere to the surface of negatively charged teeth. By introducing a negatively charged anode in the brush head and a positively charge cathode in the brush handle, the polarity is reversed allowing positively charged bacteria to be attracted to the brush head so that plaque can be more easily dislodged during brushing. Some clinical studies of such ionic therapies have demonstrated beneficial results using an ionic toothbrush.

Another challenge that people face is establishing a proper brushing routine, including brushing their teeth consistently, correctly and for a sufficient time, which is why many commercial toothbrushes incorporate brush timers which alert the end user (for example, every 30 seconds) to ensure each quadrant is brushed thoroughly. Furthermore, if an adult suffers from periodontal disease and the dentist has recommended visible therapeutic light therapy to treat this disease, it would be useful for a dentist or treating physician to monitor this activity to ensure the patient is conducting the prescribed visible therapeutic light therapy during their twice daily brushing regimen.

Thus, Applicants desire a mechanism for a safe in-home consumer device that protects the end user from these risks and allows a physician and the end user to monitor brushing behavior and progress of a prescribed therapy. WO 2015/084962 A1 discloses a light emitting toothbrush with an interlock control to prevent accidental eye exposure to the light.

### Summary of the Invention

This invention is defined in the appended claim 1, and relates to increasing the safety of a dental hygiene implement that emits visible therapeutic light, i.e. radiation in the violet and/or blue region of the visible spectrum, between 400nm and 500nm, by determining whether the implement is within the user's mouth or outside of the user's mouth. Direct eye exposure to such light can be avoided using a sensor or combination of sensors that deactivate the light source whenever it is outside the environment of the mouth and permits activation only when inside the mouth of the user.

The dental hygiene implement is a light emitting toothbrush. The light emitting toothbrush, according to the present invention, will typically further include a control circuitry which will typically be located in the handle and normally include functions such as a timer circuitry (which times the duration(s) of use of the toothbrush while brushing), an on/off duty cycle of the visible therapeutic light source or sources, a battery replacement indicator, and so on.

Preferably the driver for driving the visible therapeutic light source is equipped with constant electrical current control electronics and a suitable driver is supplied by Linear Technology, of San Jose Calif., as part no. LTC3454 which is an integrated circuit high current LED driver. The control circuitry may also include visible therapeutic light source control circuitry, which may be connected with one or more sensors located in the brush head, for detecting when the brush head is actually located within a user's mouth, thereby reducing the possibility of the visible therapeutic light being inadvertently emitted except when the toothbrush is actually located within the mouth of the user. In another embodiment, the control circuitry may alternatively include one more sensors located on the handle to determine whether the brush head is actually located within a user's mouth.

In one embodiment, an AC or DC signal loop sensor establishes a signal loop through the user's mouth to the user's hand grasping the handle of the toothbrush to verify if the toothbrush head is within the user's mouth. If the sensor establishes a signal loop, the light source remains on until the toothbrush head is removed from the user's mouth breaking the signal loop. Once the signal loop is broken, the light source may be extinguished as soon as the brush head is removed from the mouth.

In another embodiment, the dental hygiene implement uses a capacitive sensor to determine whether the implement is within the user's mouth or outside of the user's mouth. The capacitive sensor may use a current loop which detects current flowing through the body of the end user when the brush head or bristles are in contact with the mouth and the handle is in contact with the hand.

In another embodiment, the dental hygiene implement uses a capacitive displacement sensor to determine whether the implement is within the user's mouth or outside of the user's mouth. The capacitive displacement sensor can detect change of position of any conductive target such as the human body.

In another embodiment, the dental hygiene implement uses an inductive sensor to determine whether the implement is within the user's mouth or outside of the user's mouth. The inductive sensor uses an inductance loop to measure the proximity of conductors such as the human body.

In another embodiment, the dental hygiene implement uses a passive thermal infrared sensor to determine whether the implement is within the user's mouth or outside of the user's mouth. The passive thermal infrared sensor detects the warmth of the human mouth to determine whether the implement is within the user's mouth.

In another embodiment, the dental hygiene implement uses an active thermal infrared sensor to determine whether the implement is within the user's mouth or outside of the user's mouth. The active thermal infrared sensor uses a photoelectric sensor that detects reflected IR light emitted and absorbed by the sensor itself. This could be used to detect proximity inside the mouth.

In another embodiment, the dental hygiene implement uses a passive optical sensor to determine whether the implement is within the user's mouth or outside of the user's mouth.

The passive optical sensor is a light sensor that triggers the LED when it detects darkness when present inside the user's mouth. For increased sensitivity to changes in light intensity, the light sensor may be sensitive to a wavelength at least 50 nm different from the LED.

In yet another embodiment, the dental hygiene implement uses a photocell to determine whether the implement is within the user's mouth or outside of the user's mouth. A photocell is a light sensor that detects the reflection of light from a second light source on the implement when the implement is turned on. In one embodiment, the photocell may turn on the LED when it detects the reflection of light from a second light source while in the user's mouth.

In another embodiment, the dental hygiene implement uses an ultrasonic sensor to determine whether the implement is within the user's mouth or outside of the user's mouth. The ultrasonic sensor may use echo location to detect the confines of the mouth.

In another embodiment, the dental hygiene implement uses a passive optical sensor to determine whether the implement is within the user's mouth or outside of the user's mouth.

In yet another embodiment, the dental hygiene implement uses a magnetic sensor to determine whether the implement is within the user's mouth or outside of the user's mouth. In one embodiment of the present invention, the magnetic sensor may turn on the LED when it detects the proximity of metals such as the hemoglobin present in blood.

In yet another embodiment, the dental hygiene implement uses a pressure sensor to determine whether the implement is within the user's mouth or outside of the user's mouth. In one embodiment, the pressure sensor may be located under the brush head to detect movement and pressure of the brush head being pressed against the teeth. The type of pressure sensor such may be piezoelectric, cantilever switch, capacitive, potentiometric, optical or electromagnetic. In another embodiment, the pressure sensor may be located on the handle - detects torque and tension in the handle of the brush due to brushing action. The pressure sensor could be Piezoelectric, Cantilever Switch, Capacitive, Potentiometric, Optical or Electromagnetic.

In yet another embodiment, the dental hygiene implement uses a moisture sensor to detect a highly moist environment such as the mouth. This can be accomplished through various types of moisture sensors for example; capacitive or chilled mirror dew point sensors.

In one embodiment, the dental hygiene implement may include electronics having a control circuit to couple the battery and the source of light and the sensor. The sensor may have a closed state when detecting the visible therapeutic light source is inside the mouth of the user, and an open state at other times. In this embodiment, the control circuit may have four states: 1) a light off state when the sensor is in the open state and the visible therapeutic light source is off; 2) a ramp-up sequence when the sensor switches from the open state to the closed state; 3) a light on full state wherein the control circuit supplies about 100% power to the visible therapeutic light source when the sensor remains in a closed state for a duration of time; and 4) a ramp-down sequence when the sensor switches from the closed state to the open state. The control circuit may return to the light off state and cease to supply power to the visible therapeutic light source when the sensor remains in an open state for a duration of time. The control circuit may return to the light off state when the sensor switches from the closed state to the open state during a ramp-up sequence.

In another embodiment, the dental hygiene implement may further include an ionic potential in combination with a light source for use in ionic therapy. The visible therapeutic light and ionic plaque removal have entirely different modalities and in combination provide synergistic advantages above and beyond the benefits of the therapies applied individually. This is because in combination the two modalities target different strains of bacteria thus providing a more holistic approach to oral hygiene and because they affect bonding within bacterial biofilms in different ways.

In another embodiment, the toothbrush may include a communications system for exchanging data with a computing device. The toothbrush generates data about a brushing event and/or light therapy event while a person uses the toothbrush to brush his or her teeth, and the data about the event can be exchanged with the computing device. The computing device may include a tablet, desktop, laptop, tablet and smartphone, and the communications system may be a Bluetooth^{®} communications module that pairs to the computing device using a Bluetooth^{®} protocol. The toothbrush may exchange data with the computing device while paired, and may store data when the computing device is not within a range to be paired with the toothbrush. Once the computing device is within range for pairing, the toothbrush can upload the stored data to the computing device. The data exchanged between the toothbrush and the computing device may include brushing and light therapy habits of the user.

A software application may be installed on the computing device for modifying the settings of the toothbrush and analyzing brushing and light therapy habits of the user. For example, modification of the settings of the toothbrush may include modifying the intensity of the light source. Similarly, analyzing the brushing and light therapy habits of the user may include displaying analyzed results on the computing device. For instance, the analyzed results may include the user's average brushing time and average number of brushes per day. The analyzed results may be shared to a second computing device. Data on the number of joules (or other measures) of light energy may also be collected, analyzed, transmitted, stored and/or displayed.

In another embodiment, the toothbrush may include a second light source that emits light with a wavelength in the range of about 600 nm to about 1000 nm, wherein the second light source is activated when the second light source is in the mouth of the user and the sensor has a closed circuit forming a signal loop.

In another embodiment, the toothbrush may include a light assembly comprising a light source with a light source concentrator installed within the handle. The light source concentrator directs light through a light pipe installed on the distal end and emits light out of an aperture. One example of a light source concentrator is a parabolic concentrator. The parabolic concentrator can emit light at an angle of incidence to create total internal reflection within the light pipe, thereby enabling light to travel along the light pipe while minimizing losses. In one embodiment, the light pipe and light source concentrator are less than about 8 mm in diameter. A concentrator may not be needed if the LED light source were replaced with an unfocussed laser diode with a sufficiently narrow inherent beam width. The light source may be activated by the current signal loop discussed herein, or other sensors as discussed herein.

In certain embodiments, the light source of the light assembly may comprise an array of LEDs on a PCB board. The light source may emit at a wavelength in the range of 400 nm to 500 nm. The toothbrush may further include a beam-steering component to direct emitted light from the light source concentrator to the aperture; for example, a lens. In some instances, the light pipe may comprise a curved surface to guide the emitted light to the aperture. In other instances, the light pipe may comprise a non-conductive plastic.

In another embodiment, the toothbrush handle and the distal end are separable, and the light pipe may align with the light source concentrator when the distal end is mounted on the handle. For example, the distal end may mount on the handle with a bayonet mount. In another example, the distal end mounts on the handle with a press fit. In another example, the handle has a female fitting light source concentrator and the distal end mounts on the handle by insertion in the female fitting. Another example may include an embodiment wherein the handle has a male fitting light source concentrator and the distal end mounts on the handle by insertion of the handle into a female fitting on the distal end.

Other embodiments of the present invention may include combinations of two or more of these sensor types as disclosed above. Furthermore, the sensor examples listed above is not intended to be a complete list of the sensors available for use with the present invention. Therefore, the present invention is not to be limited to the use of the specific sensors or oral care instruments described herein.

### Brief Description of the Drawings

The invention will be better understood by a reading of the Detailed Description of the Examples of the Invention along with a review of the drawings, in which:
Figure 1 shows the completion of a signal loop by a user inserting a toothbrush into his mouth;
Figure 2 depicts a representative oral care instrument, a toothbrush, illustrating various aspects;
Figure 3 depicts an alternate embodiment in which the wet bristles of the brush head are conductive to form a contact for completion of the signal loop;
Figure 4 shows a toothbrush with conductive metal pads;
Figure 5 shows a toothbrush with conductive plastic pads;
Figure 6 is a schematic of an exemplary control circuit;
Figure 7 is a schematic of an exemplary oral care instrument that has a replaceable head and neck;
Figure 8 is a schematic of an exemplary oral care instrument that has a replaceable brush head;
Figure 9 is an exploded view of a toothbrush with a capacitive sensor;
Figure 10 is a circuit view of the embodiment shown in Figure 9;
Figure 11 is a partial cross-sectional view of an embodiment of the invention where the sensor is a capacitive displacement sensor placed on the handle;
Figure 12 is a circuit view of an embodiment of the invention where the sensor is a capacitive displacement sensor;
Figure 13 is depicts an alternate embodiment where the sensor is an inductive proximity sensor;
Figure 14 shows an embodiment where the sensor is a passive thermal sensor placed near the upper portion of the handle of a toothbrush;
Figure 15 shows an embodiment where two passive thermal sensors are placed near the upper portion of the handle of a toothbrush;
Figure 16 shows an embodiment where the sensor is a passive thermal sensor placed on the back of the bristle plate of a toothbrush;
Figure 17 shows an embodiment where the sensor is an active thermal infrared sensor placed on the back of the bristle plate of a toothbrush;
Figure 18 shows an embodiment where the sensor is a passive optical sensor placed on the back of the bristle plate of a toothbrush;
Figure 19 shows an embodiment where the sensor is a photocell placed on the brush head of a toothbrush;
Figure 20 shows an embodiment where the sensor is an ultrasonic sensor placed on the brush head of a toothbrush;
Figure 21 is a partially exploded view of another embodiment of the present invention, where the sensor is a pressure sensing system;
Figure 22 is a side view of a portion of the pressure sensing system of Figure 21;
Figure 23 is a partial fragmentary perspective view of another embodiment of the invention where the oral care instrument is a toothbrush having a Hall effect sensor;
Figure 24 is a partial fragmentary perspective view of a toothbrush in accordance with another embodiment of the present invention;
Figure 25 depicts a toothbrush illustrating various aspects of another embodiment of the present invention wherein the sensor is a moisture sensor;
Figure 26 depicts another embodiment of a toothbrush wherein the sensor is a moisture sensor;
Figure 27 shows an embodiment of the present invention wherein the sensor is an accelerometer;
Figure 28 shows an embodiment of a toothbrush including a fingerprint sensing module;
Figure 29 is a flowchart illustrating an embodiment of steps for use of a toothbrush having a fingerprint sensing module and a plurality of LED status indicators;
Figure 30 is a simplified schematic of an embodiment of a toothbrush including a fingerprint sensing module and a plurality of LED status indicators;
Figure 31 illustrates an interlock sensor software state transition diagram;
Figure 32 illustrates one example of a ramp-up sequence in accordance with one embodiment;
Figure 33 is a table showing various sensor types compatible with the interlock sensor software and examples of triggering conditions;
Figure 34 is a schematic view of a toothbrush according to one embodiment wherein the interlock sensor is a current loop sensor with a cathode provided in the handle and an anode provided in the brush head for ionic therapy;
Figure 35A is a schematic view of a toothbrush according to another embodiment wherein the toothbrush includes a communication module;
Figure 35B is a schematic view of a toothbrush according to another embodiment wherein the toothbrush includes a first light source and a second light source;
Figure 36 is an enlarged schematic view of a toothbrush according to another embodiment wherein the light source is installed on the handle of the toothbrush;
Figure 37 is a table with an accompanied schematic of a light source concentrator disclosing how the angle of incidence varies with the size of the light source concentrator;

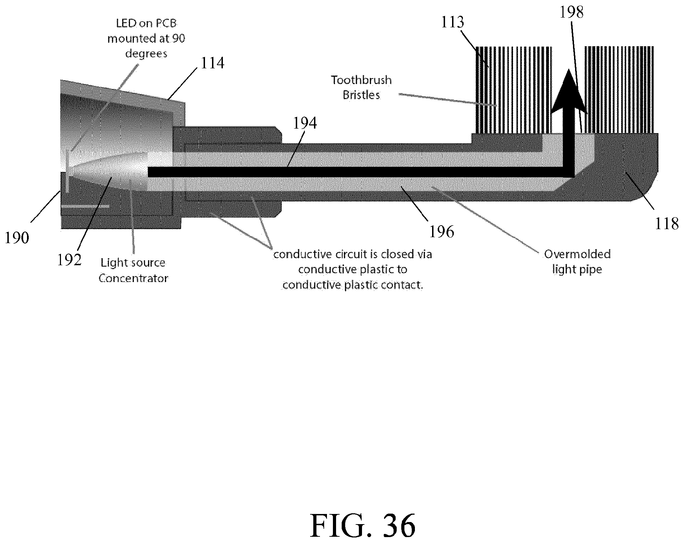

Figure 36 is an exploded view of the light assembly in Figure 81;

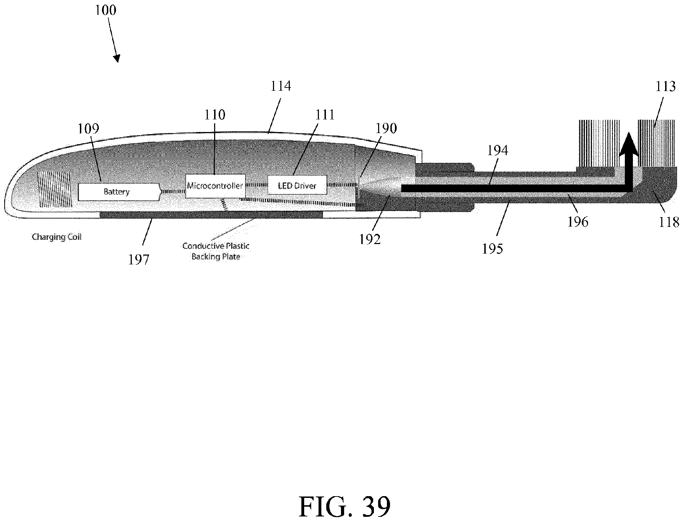

Figure 39 is a schematic view of a toothbrush having a conductive plastic backing plate on the handle and a brush head comprising a conductive plastic with a light source installed on its handle;

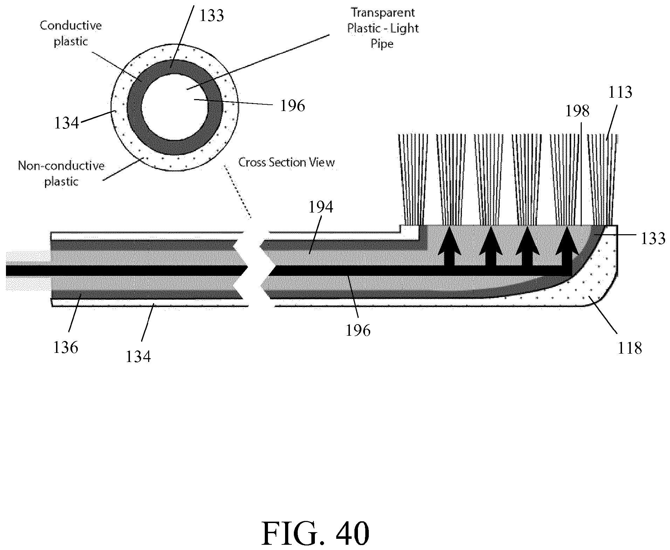

Figure 40 is an enlarged schematic view of another embodiment wherein a light pipe is encased in conductive plastic and surrounded by a non-conductive plastic layer forming brush head; and

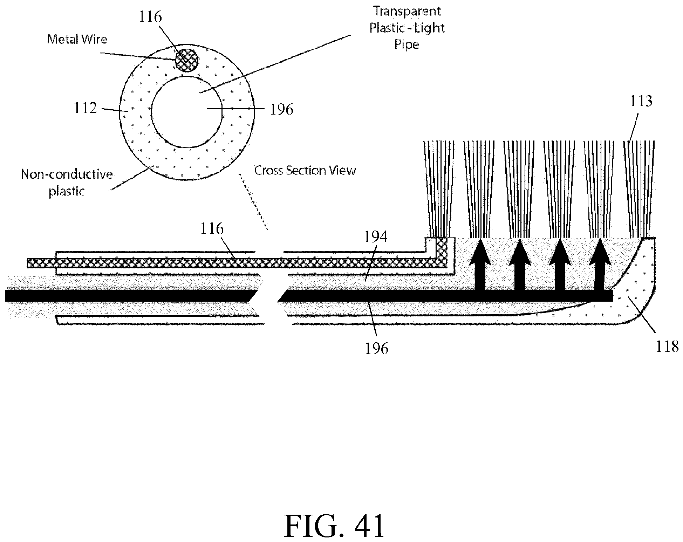

Figure 41 is an enlarged schematic view of another embodiment wherein a light pipe is encased in non-conductive plastic and the toothbrush includes a metal wire for use as part of a sensor; and

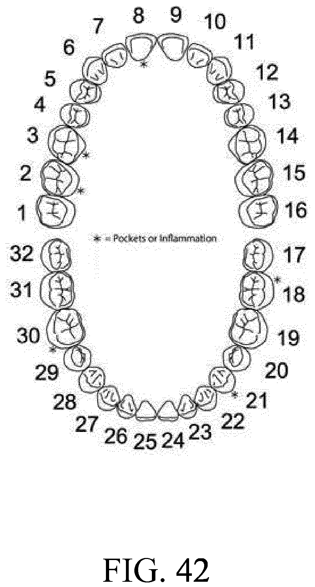

Figure 42 is an example of a dentist's universal teeth numbering system chart.

### Detailed Description of Examples of the Invention

Exposing the mouth to light in the visible therapeutic light (i.e. violet and/or blue region of the visible spectrum, between 400nm and 500nm) can be useful for a variety of purposes, including destroying bacteria and accelerating the whitening effects of a tooth bleaching agent. However, direct eye exposure should be limited in order to prevent eye damage. Thus, an aim of the present invention is to determine whether an oral care instrument is within the user's mouth or outside of the user's mouth. If the instrument is within the user's mouth, then a light source such as an LED is turned on to emit light. However, if the instrument is outside the user's mouth, the LED shuts off to prevent light exposure to the eyes. As used herein LED includes LED arrays unless otherwise specified. Laser diodes can also be used within the scope of the invention.

Aspects of the invention are illustrated in the remainder of this disclosure with reference to a manual or electric motorized toothbrush, although it is understood that the operation of any number of light-emitting oral care instruments, together with the associated advantageous features and/or beneficial effects described herein, could likewise be achieved. Other oral care instruments may include those used in dental curing lamps, oral flossing implements, water-based flosser, hand-held blue light facial acne treatment devices, and oral surgical instruments, etc.

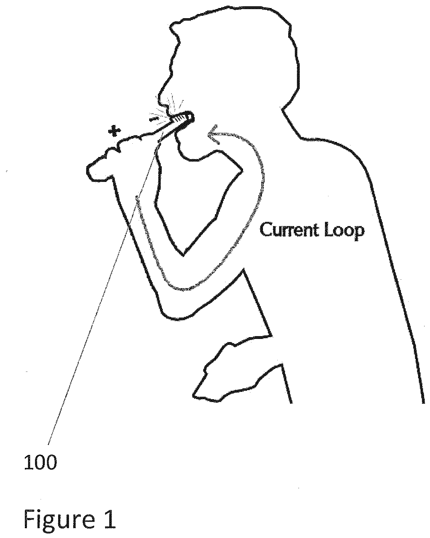

In one embodiment, as shown in Figure 1, a light-emitting oral care instrument 100 activates upon the completion of an electrical circuit between the brush handle in contact with the user's hand and contact of the brush head with the user's mouth.

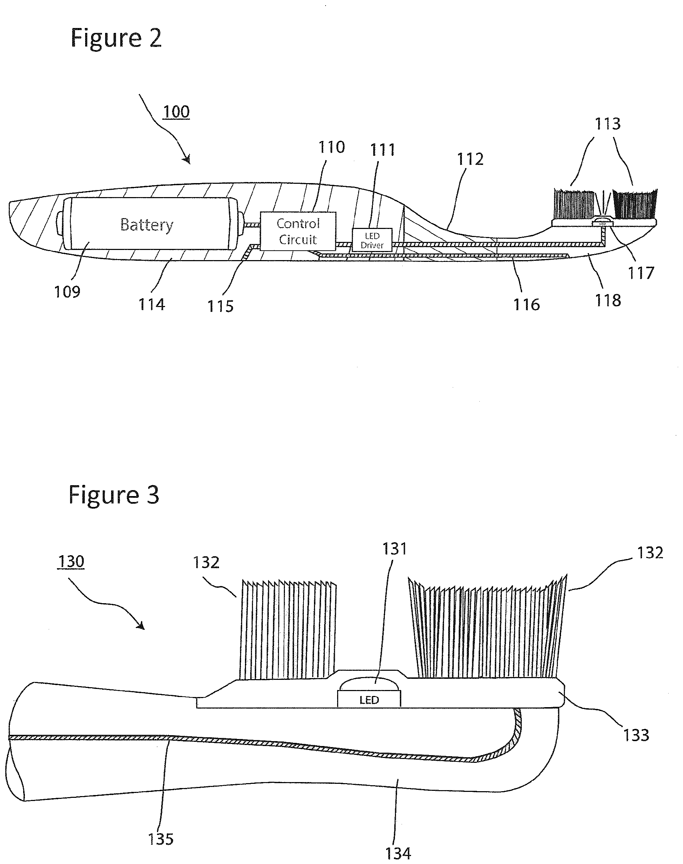

In the embodiment of the light-emitting toothbrush 100 shown in Figure 2, the brush head 118 and the handle 114 are injection molded of an electrically-conductive plastic with a non-conductive hydrophobic plastic spacer 112 located between the brush head 118 and the handle 114. The nonconductive plastic spacer 112 electrically insulates the brush head 118 and the handle 114 from each other. The brush head 118 and handle 114 are electrically connected, via a lead 116, to a control circuit 110 that can produce a low level electrical signal. While DC current is likely easiest with a battery source, the signal could be AC. The control circuit 110 can include a high sensitivity current sensor, such as Linear Technology LTC1440 Ultralow Power Single/Dual Comparator with Reference located on the control circuit 110. That comparator is available from Linear Technology Corporation, 1630 McCarthy Blvd., Milpitas, CA 95035-7417 1 (408) 432-1900, linear-tech.com.

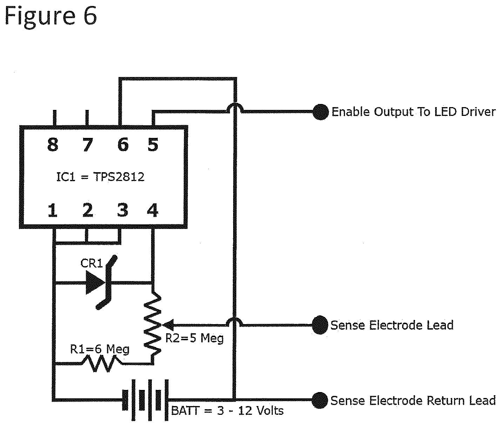

Alternatively, the signal loop sensor circuit can be the one seen in Figure 6, implemented with off the shelf components. An integrated circuit (IC1) shown in Figure 6 can be a TPS2812 Dual High-Speed Mosfet Driver available from Texas Instruments, Inc. of Dallas, Texas. One of the sensor electrodes 116 is connected through a battery supplying from three to twelve volts, to pins 1-3 and 6 of IC1. The other sensor electrode 115 is connected across and adjustment bridge variable resistor R2 and to pin 4 of IC1 across resistor R1 to pins 1-3. Pin 4 is tied through Zener diode CR1 to pins 1-3. Pin 5 of IC1 is tied as the output to the LED driver. When the signal loop through electrodes 115 and 116 is completed, the current is sensed in IC1, outputting a signal on pin 5 to activate the driver for the LED.

Prior art toothbrush sensors were implemented with older bipolar transistor technology and required high currents in the signal loop and higher voltages, producing undesirable tingling sensations for the user. The preferred signal loop sensor uses ultra-low-power CMOS technology to reduce the detection current threshold to a sub-microampere level at around one volt of potential difference, preventing any tingling sensation. Another advantage is an ultra-low battery consumption current.

A signal loop is formed through the body of the user by holding the brush handle 114 and placing the brush head 118 or wet bristles of the brush 113 in contact with the mouth. A voltage across the handle and the brush head results in a small signal current that flows through the loop and is detected by the current sensor. The current sensor outputs a signal through the control circuit 110 to the LED driver 111, which delivers current to the LED 117 within the user's mouth, causing the LED 117 to illuminate the mouth of the user. The LED preferably emits short wave length light in the band between 400-500 nm, more preferably 400-450 nm, and more preferably yet of 400-420 nm.

In an alternate embodiment 130 shown in Figure 3, conductive plastic is used in the part 133 of the brush connecting the bristles 132 to a sheet of conductive plastic 133, instead of the entire brush head. Alternatively, but also possibly in combination, as shown in Figure 3, the bristles 132 of the brush may also be conductive. When wet bristles 132 come in contact with the mouth of the user during normal brushing operations a circuit is formed via a sense electrode 135 connected to the control circuit 110, which can be used to detect the completion of the electrical signal loop and signals the driver 111 to supply electricity to the LED 131. The remainder of the brushhead 134, is composed of non-conductive plastic.

Typically when brushing teeth, a user will grasp the handle 114 of toothbrush 100, apply toothpaste to the bristles 113 and place the brush head 118 in the mouth and proceed to brush their teeth. While the toothbrush handle 114 is in contact with the user's hand and the brush head 118 is located in the mouth of the user, contact between the electrically conductive plastic of the brush head 118 and mouth of the user completes the signal loop, enabling a small DC or AC current (sensor current) to be initiated and detected by the control circuit 110. The receipt of the sensor current is signaled to the LED driver which, in turn, controls illumination of the LED 117, in this case, turns on the LED 117. When contact between the brush head 118 and the mouth is broken, i.e., when the brush head 118 is withdrawn from the mouth, the signal loop is broken, the flow of sensor current stops, and the LED 117 is turned off. It is to be appreciated that the sensor current required to control the LED 117 will preferably be in the 10 and 100 nanoampere range and would only be detectable with a high sensitivity current meter associated with the control circuitry 110. Due to the use of such a low electrical current, the user will be safe from harm and will not be exposed to danger. The galvanic current generated when a person touches a wet metal object is far greater than the current used in our application, so the current passing through the user's body is harmless and imperceptible.

The electronics, i.e. the LED driver and control circuitry 110 are configured to regulate or adjust the sensor current so that the brush head 118 must be in contact with the user's mouth to turn on the LED 117. Merely contacting the dry skin from hand to hand will not turn the LED 117 on since the dry hand is not as conductive as the wet mouth. This reduces the possibility of turning the LED 117 on merely by handling the toothbrush 100 with bare hands and inadvertently activating the LED 117 while the brush head 118 is not in the user's mouth.

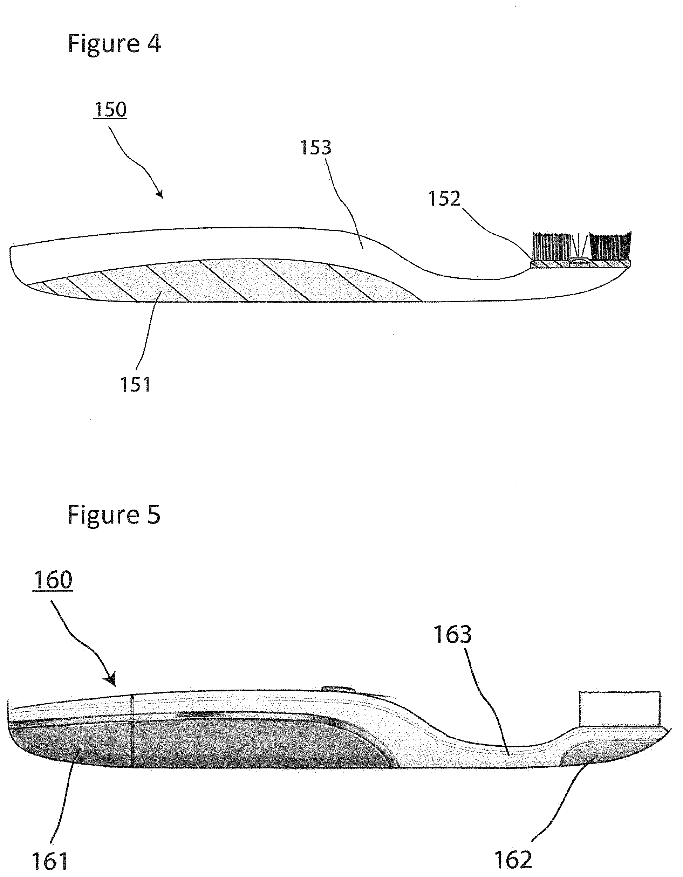

In a further embodiment of the toothbrush 150 as illustrated in figure 4 which is similar to the prior embodiment, the brush head and handle 153 could be formed from a hydrophobic, nonconductive plastic instead of the electrically conductive plastic. In this embodiment, electrically conductive metal pads 151, 152 are located on the exterior surface of the toothbrush 150. One or more metal pads 151 are located on the handle 112' and one or more metal pads 152 are located on the brush head. The metal pads 151 are positioned on the toothbrush 150 for optimal contact with the skin of the hand and pad 152 is mounted for contact with the mouth or bristles of the toothbrush. When the wet tips of the brush bristles are in contact with incisor teeth or mouth and the pad 151 is in contact with the user's hand, an electrical circuit is formed through the body of the user. It is to be understood that the metal pads 152 of the brush head should be spaced apart from the metal pads 151 of the handle.

In use, while the metal pads 151 of the toothbrush handle are in contact with the user's hand and when the brush head 152 is located in the mouth of the user or wet bristles are in contact with any wet portion of the mouth or teeth, contact between the metal pads 152 of the brush head and mouth of the user completes a signal loop, enabling a small DC current (sensor current) to be initiated and detected by the control circuit **110.** The sensed current causes the control circuit to activate the LED driver to turn on the LED 126. When contact between the brush head and the mouth is broken, i.e., when the brush head is withdrawn from the mouth, the signal loop is broken, the flow of sensor current stops, and the control circuit turns off.

A further embodiment 160 is shown in Figure 5. The handle has a portion 161 of conductive plastic, and the head has a portion 162 of conductive plastic. Portion 163 is non-conductive. The conductive plastic areas are connected internally to circuits as described in connection with Figure 2 and so the toothbrush of Figure 5 can be used in like manner to the toothbrush of Figure 2.

Many electric toothbrushes employ a timer to alert the user of the end of a preset brushing time, for example, two minutes, as recommended by the American Dental Association. These timer circuits are commonly combined with vibration or noise to alert the end user to the completion of a recommended brushing period. The signal loop sensor disclosed herein can be combined with such a timing circuit in a manner that causes elapsed time to be recorded only when the sensor is activated (i.e. light is on). This would facilitate the assurance that the brush timer was actually measuring elapsed time in the mouth of the user and not simply elapsed time of the manual activation of the brush. The control circuit can also include a timer to turn off the LED at a preset time, such as two minutes, signaling to the user that tooth brushing has lasted two minutes. Other ways to signal the user of the completion of two minutes can be substituted.

A conventional make/break switch can be included in one of the conductors 115 or 116 or elsewhere in the electrical circuit, if desired. Thus, the signal loop is completed only if the user closes that switch AND inserts the toothbrush head into the mouth. This can provide a further safety and convenience feature. Alternatively, a make/break switch could be located elsewhere in the circuit from the battery, to the control circuit through the LED driver to LED.

Since the LED will be on only when the light from the LED is safely and effectively used, the life of a charge on the battery should be longer.

The battery can be rechargeable or replaceable, as will be apparent to those of ordinary skill in the art. The toothbrush can be a power toothbrush such as a vibrating, sonic or spin brush or a manual toothbrush.

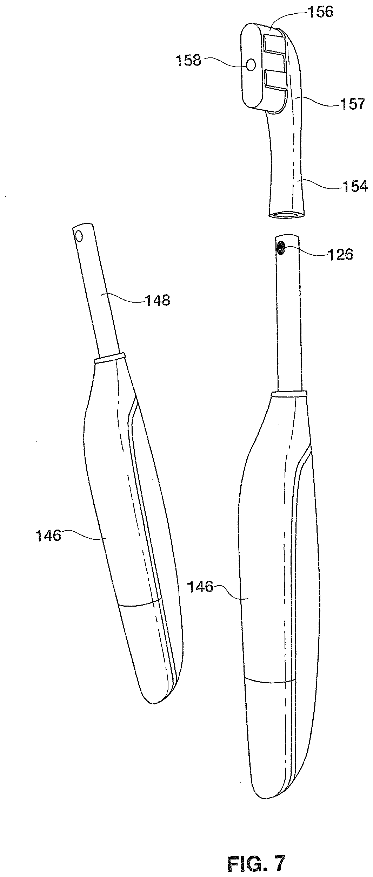

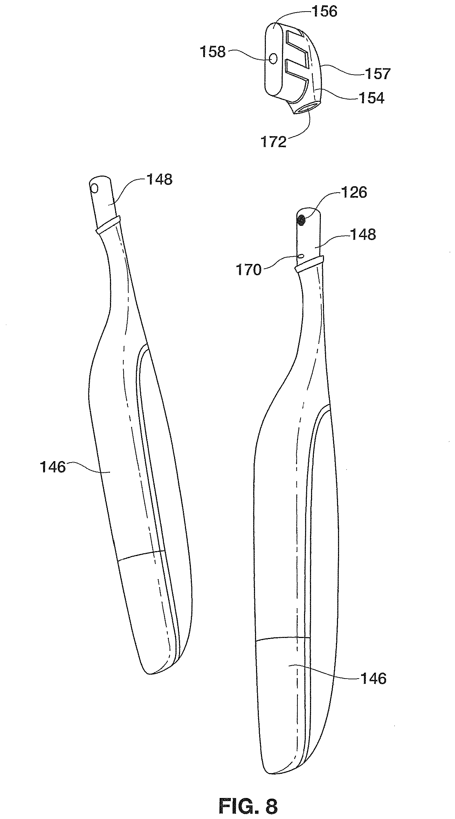

The brush head and/or neck can be replaceable, as long as the replacement part has the correct electrical contact and a conductor that can reliably connect to a mating conductor in the brush handle. The design can allow the sensor current to flow across the junction of the permanent part and the replaceable part through either two sheets of conducting plastic or a metal connector such as a pin connector. Examples are seen in Figures 7 and 8.

As seen in Figures 7 and 8, a brush handle 146 is provided having the necessary battery, charging electronics and LED, all self-contained and water-tightly encapsulated within the handle. The handle includes a stem 148 in which the LED 126 is mounted. Figures 7 and 8 show two different embodiments, with the stem 148 in Figure 7 being longer than the stem 148 of Figure 8. The replaceable brush head 157 has a hollow shaft 154 of an internal diameter slightly larger than the diameter of the stem 148, so the stem 148 can be inserted into the hollow shaft 154. The brush head has an array of bristles 156 and an opening 158 to allow the light from the LED 126 to pass toward the teeth as they are brushed by the bristles. Preferably the stem and hollow shaft have complementary, non-circular shapes so that the bristles do not rotate around the stem, but stay in a fixed orientation. When the bristles are spent, the brush head 157 can be removed from the stem 148 and replaced with a new brush head. Other appliances appropriate for a tooth whitening brush pattern, light curing of gum infections, tongue scraper, flosser, etc can be configured with similarly shaped hollow shafts so they can also be mounted onto the stem 148.

In the embodiment shown in Figure 7 the plastic of the stem 148 and the head 157 are made electrically conductive, so that the signal loop can be completed as the user puts the head 157 (as installed on the stem 148) into his or her mouth and grasps the handle 146.

In the embodiment shown in Figure 8 a conductor in the plastic of the stem 148 has an electrical contact 170; the head 157 has an electrical contact 172 within the shaft 154 positioned to mate with contact 170 when the head is mounted on the stem. The signal loop can be completed as the user puts the head 157 (as installed on the stem 148) into his or her mouth and grasps the handle 146.

A further enhancement of the interlock control feature of this toothbrush can be realized using an AC control signal in place of the DC loop sensor current. A low-frequency 200 Hz to 10 kHz square or sinusoidal waveform AC signal with a peak to peak voltage of no greater than 1 V will be applied to the toothbrush handle. This AC signal will be conducted through the user's body in much the same way as a DC current, as described above. One way to implement this is through selective tone filtering in which a Controlled Oscillator in the handle puts off a square wave form in the low audio band of 1 to 2 kHz. A narrow band tone filter only allows one tone to come through in the brush head. This Selective Tone Filter in the brush head is looking for one certain tone. When it receives that tone, it turns on the LED.

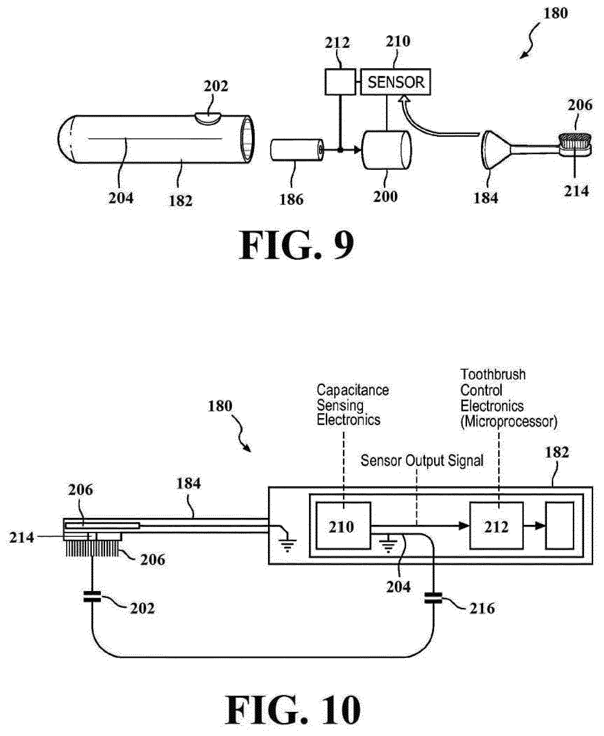

In another embodiment of the present invention, as shown in Figures 9 and 10, the sensor used to detect whether the oral care instrument 180 is within the user's mouth is a capacitive sensor. The oral care instrument may be a toothbrush 180 with a battery 186, a LED driver 200, and an on/off switch 202. An electronics ground 204 is located on the handle 182 of the brush. A conductive electrode sensing element 206 is installed preferably on the brush head 184. A sensor system 210 measures the capacitance 216 between the sensing element 206 and the electronics ground 204. A microprocessor/controller 212 determines whether the measured capacitance 216 is above a threshold value, which indicates that the toothbrush 180 is inside the user's mouth. If the measured capacitance exceeds the threshold value, then the LED driver 200 receives a signal and turns on the LED 214. If the measured capacitance drops below the threshold value, then the LED driver turns off the LED 214.

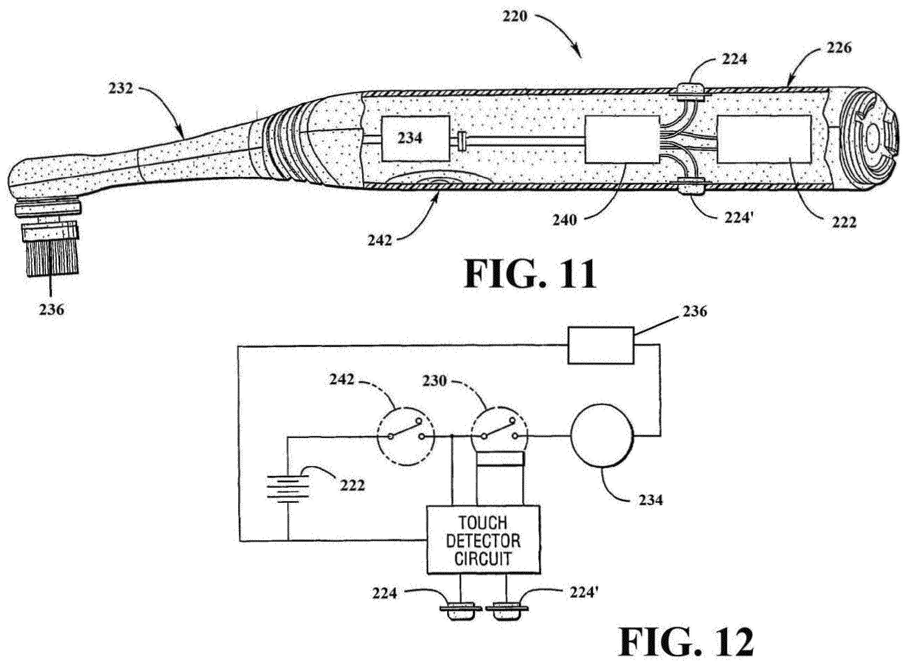

In yet another embodiment, the sensor of the present invention may be a capacitive displacement sensor. One or more tactile sensors are preferably placed on the handle portion of the oral care instrument, and measure capacitance of a conductive target such as the human body. As shown in Figure 11, the oral care instrument is a toothbrush 220 with a battery 222 and tactile sensors 224, 224' placed on opposing sides of the handle 226. A switch 230 is off when in its first position. When a user grips the handle 226 at both tactile sensors 224 and 224', preferably when the brush head 232 is inserted inside the user's mouth, the capacitive sensor 240 senses an increase in capacitance. As shown in Figure 12, the capacitive sensor 240 causes switch 230 to move to its second position, signaling the LED drive 234 to turn on the LED 236. An additional on/off switch 242 operated by the user may be installed onto the oral care instrument.

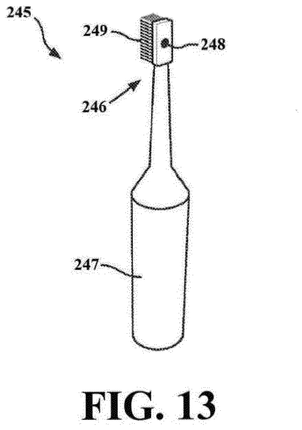

In another embodiment of the invention, the sensor is an inductance proximity sensor which uses a radio frequency loop to sense the proximity of conductors such as the human body. In one embodiment, as shown in Fig. 13, the dental hygiene implement is a toothbrush 245 having a brush head 246 and handle 247. In this embodiment, the proximity sensor 248 is on the back of brush head 246. The inductance proximity sensor 248 is comprised of an LC oscillating circuit with a signal evaluator. The LC oscillating circuit is comprised of a coil and a capacitor that emits a high-frequency electromagnetic alternating field at the sensing face of the sensor. When the brush head is inserted into the user's mouth, Eddy currents are generated that reduces the oscillations within the LC oscillating circuit. The signal evaluator detects this change in oscillation frequency, and the resulting reduction in oscillations sends a signal to the LED driver to turn on LED 249. When the brush head is removed from the user's mouth, the oscillations from the LC oscillating circuit return to normal and the LED driver turns LED 249 off. Different types of inductor circuits may be used in other embodiments of the present invention.

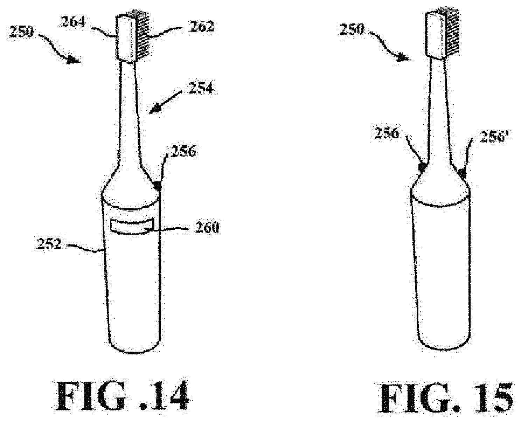

In another embodiment of the invention, the sensor is a passive thermal infrared sensor. The passive thermal infrared sensor may be placed anywhere on an oral care instrument. In the embodiment shown in Figures 14 and 15, the oral care instrument is a toothbrush 250 with a handle 252 and brush head 254. At least one passive thermal infrared sensor 256 is placed near the upper portion of the handle 252 toward the brush head 254. As the user inserts the brush head into the mouth, the microprocessor 260 detects a change in infrared light as detected by the passive IR sensor 256. The resulting increase in IR light causes the microprocessor 260 to turn on LED 262. As the user removes the brush head out of the mouth, the resulting decrease in IR light sensed by the passive IR sensor 256 causes the microprocessor 260 to turn off LED 262.

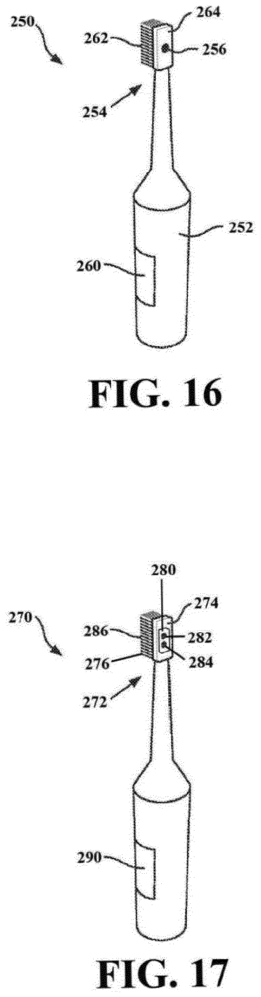

In another embodiment, the passive thermal infrared sensor is placed on the brush head as opposed to the handle. In the embodiment shown in Figure 16, the passive IR sensor 256 is placed on the back of the bristle plate 264. Like the embodiment in Figures 14 and 15, if the user places the brush head in his mouth, then the microprocessor 260 will turn on LED 262 due to the increase in IR light as sensed by passive IR sensor 256. As the user removes the brush head out of the mouth, the resulting decrease in IR light sensed by the passive IR sensor 256 causes the microprocessor 260 to turn off LED 262. The change in infrared light as the brush head enters the mouth is understood to be a result of the change in temperature. Thus, it is understood that the passive IR sensor disclosed herein may be substituted with other means for detecting changes in temperature.

In another embodiment of the invention, the sensor is an active thermal infrared. This sensor uses a photoelectric sensor that detects reflected IR light emitted and absorbed by the sensor itself. This could be used to detect proximity inside the mouth. The active thermal IR sensor 280 is typically comprised of a light source 282 that emits IR light and a photo diode 284 that detects changes in the sensing environment as the IR emitted from the light source is redirected. In one embodiment, an active thermal infrared sensor is placed on the brush head 272 of a toothbrush 270. The active thermal infrared sensor may be placed either on the front face of the plate with the bristles 276 and LED 286. Alternatively, as shown in the embodiment in Figure 17, the active thermal infrared sensor 280 may be placed on the back of the bristle plate 274. If the user places the brush head in his mouth, then the microprocessor 290 will turn on LED 286 due to the change in IR light as sensed by photo diode 284. As the user removes the brush head out of the mouth, the IR light returns to a default value as detected by photo diode 284 which causes the microprocessor 290 to turn off LED 286.

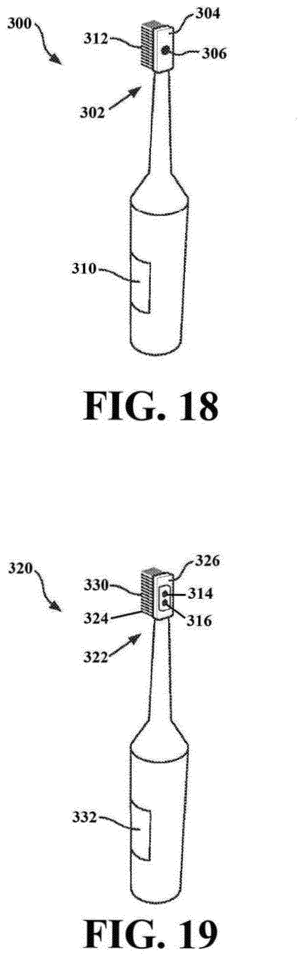

In another embodiment of the invention, the sensor is a passive optical sensor where the darkness caused by inserting the oral care instrument into the mouth triggers the short wavelength LED to turn on. Figure 18 shows an embodiment wherein the passive optical sensor is installed on a toothbrush 300. In this embodiment, the passive optical sensor 306 is preferably on the brush head 302 of the toothbrush. In the embodiment shown in Figure 18, the passive optical sensor 306 is placed on the back of the bristle plate 304. If the user places the brush head in his mouth, then the microprocessor 310 will turn on LED 312 due to the decrease in light as sensed by passive optical sensor 306. As the user removes the brush head out of the mouth, the resulting increase in light sensed by the passive optical sensor 306 causes the microprocessor 310 to turn off LED 312.

In another embodiment of the invention, the sensor is a photocell 314 that detects the reflection of light from a second light source 316 on the oral care instrument. In one embodiment, a photocell is placed on the brush head 322 of a toothbrush 320. The photocell 314 and accompanying light source 316 may be placed either on the front face of the plate with the bristles 324 and LED 330. Alternatively, as shown in the embodiment in Figure 19, the photocell 314 and secondary light source 316 may be placed on the back of the bristle plate 326. If the user places the brush head in his mouth, then the light emitted by light source 316 will reflect and be detected by the photocell 314. As the reflected light is detected by photocell 314, the microprocessor 332 will turn on LED 330. As the user removes the brush head out of the mouth, the photocell 314 will no longer detect the reflected light which causes the microprocessor 332 to turn off LED 330.

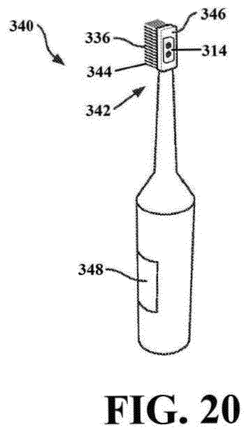

In another embodiment of the invention, the sensor is an ultrasonic sensor that emits sound waves and uses echo location to detect whether the oral care instrument is within the confines of the mouth. In one embodiment, an ultrasonic sensor 334 is placed on the brush head 342 of a toothbrush 340. The ultrasonic sensor 334 may be placed either on the front face of the plate with the bristles 344 and LED 336. Alternatively, as shown in the embodiment in Figure 20, the ultrasonic sensor 334 may be placed on the back of the bristle plate 346. If the user places the brush head 342 in his mouth, then the duration that it takes for the sound emitted by ultrasonic sensor 334 to be received back will decrease. The decrease in durational response will cause the microprocessor 348 will turn on LED 336. As the user removes the brush head out of the mouth, the duration that it takes for the sound emitted by ultrasonic sensor 334 to be received back will increase causing the microprocessor 348 to turn off LED 336.

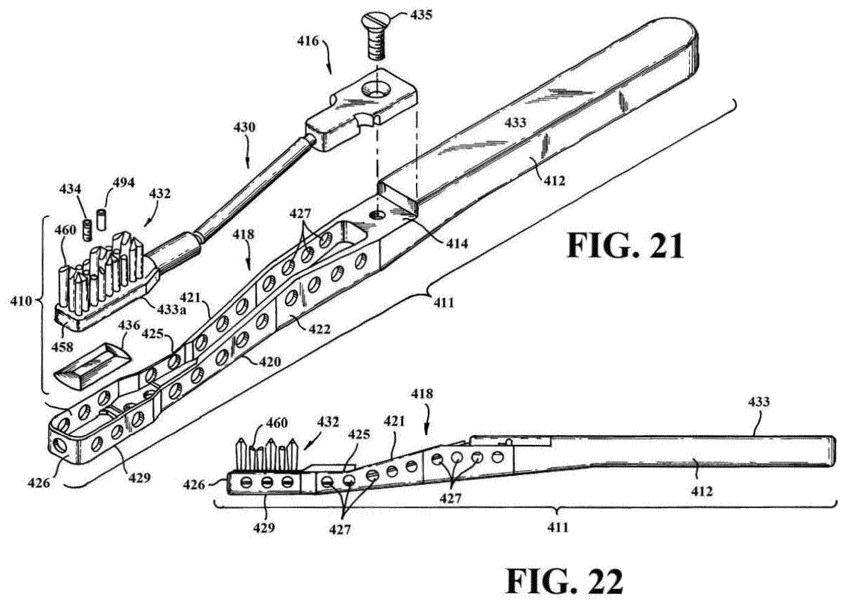

In another embodiment of the invention, the sensor is a pressure sensor under the brush head that detects movement and pressure of the brush head being pressed against the teeth. Figure 21 shows the pressure sensing system 410 of the present invention 410 and a toothbrush body referred to generally at 411. Generally, toothbrush body 411 will be a manual toothbrush. The toothbrush body 411 includes a handle portion 412, which is configured to be grasped by the hand of the user. In the embodiment shown, handle 412 is approximately 3.75 inches long, while the entire toothbrush body 411 is approximately 7.5 inches long. In the embodiment shown, toothbrush body 411 is made of a filled nylon, but could be other materials as well, including polypropylene and other plastics. Handle portion 412 is in the embodiment shown approximately 0.5 inches wide and approximately ⅜-inch high. The handle portion 412 is closed about all four sides and its two ends.

At the distal end of handle 412 is a portion 414 which in the embodiment shown is adapted to receive a hinged member portion 416 of the pressure sensing assembly 410. The remaining portion of the toothbrush body is referred to at 418, and is generally U-shaped in cross-section, open at the top. The remaining portion 418 comprises a base 420, two upstanding sides 421, 423 and a forward end wall 426. This arrangement provides rigidity for the toothbrush body. From the receiving portion 414, toothbrush body 411 begins to taper inwardly at both sides over a short distance until the width of the toothbrush body is approximately 0.25 inches. Over this distance, the top edges of the sides 421, 423 toothbrush body are flat for a small distance and then angle downwardly until point 425 on the toothbrush body. Over this distance, base 420 angles slightly downwardly. The drawings show this structural arrangement, in particular Figure 22.

From point 425 to forward end wall 426, the toothbrush body is flat and is adapted to receive a conventional toothbrush brushhead 432. The distance from the lower surface 429 of the flat section 431 to the upper surface 433 of handle portion 412 is approximately 0.75 inches, while the height of the toothbrush body in the flat section 431 is approximately 0.28 inches.

The sides 421 and 423 and the base 420 over the length of the toothbrush body from receiving portion 414 to the forward end wall 426 have a plurality of openings 427-427 therethrough. In the embodiment shown, these openings are circular, approximately 0.125 inches in diameter, spaced approximately 0.25-0.35 inches apart. In base 420 of flat section 431 is an elongated slot 437, which is discussed in more detail below. The openings could have other shapes and spacing, however. The use of openings, with an entirely open top, has several advantages. It allows fluid to easily escape the brush, without trapping oral tissue in the openings. This arrangement further permits the use of the hinged arm pressure sensing assembly 411 without the use of seals between the arm and the body. The openings further are large enough to not only allow rinsing water to move freely in and through the toothbrush body during cleaning, but also allows the unit to dry out thoroughly between uses.

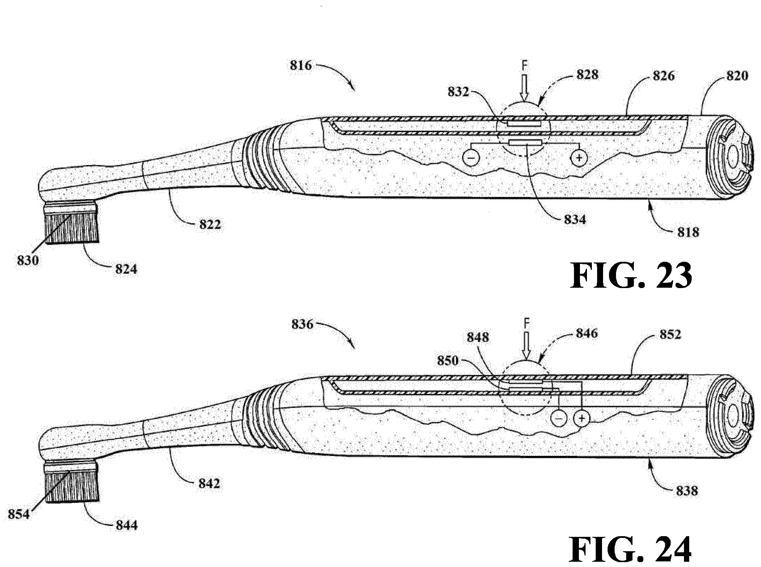

Other types of switches may be used with a toothbrush having a compressible portion, two of which are shown in FIGS. 23 and 24. FIG. 23 shows a toothbrush 816 comprising a toothbrush body 818 including a handle portion 820 and a brush head portion 822, including bristles 824. The handle portion 818 includes a compressible portion 826. A switch 828 is disposed in relation to the compressible portion 826 such that compressing the compressible portion 826 actuates the switch 828, which activates light sources 830. The switch 828 comprises a magnet 832 and a Hall effect sensor 834. The magnet 832 is located beneath the compressible portion 826 such that application of a force (F) to the compressible portion 826 causes the distance between the magnet 832 and the Hall effect sensor 834 to decrease. When this distance is small enough, current flows through the Hall effect sensor 834 and the light sources 830 are activated.

Another type of switch that can be used in conjunction with a compressible portion on a toothbrush handle is shown in FIG. 24. A toothbrush 836 comprises a toothbrush body 838 including a handle portion 840 and a brush head portion 842, including bristles 844. A switch 846 comprises first and second contact plates 848, 850 disposed in relation to a compressible portion 852 of the handle portion 838 such that compressing the compressible portion 852 causes the two contact plates 848, 850 to move closer to each other until they contact, thereby actuating the switch 846 and activating light sources 854.

In another embodiment of the invention, the sensor is a moisture detection - uses a moisture sensor to detect a highly moist environment such as the mouth. This can be accomplished through various types of moisture sensors for example; capacitive or chilled mirror dew point sensors. Such a sensor is disclosed in US 2009/0087813 to Cai et al.

These motion sensor embodiments of the present invention relate to a bio-active oral care instrument, having the ability to active an LED automatically, when the instrument or a portion thereof is exposed to one or more conditions, such as the ambient electrical conductivity, existing in the oral environment. Other conditions and combinations of conditions, such as pH, temperature, solute concentrations, etc. could likewise be detected and used as the basis for automatic operation. Furthermore, aspects of these embodiments are illustrated in the remainder of this disclosure with reference to an electric motorized toothbrush, although it is understood that the operation of any number of oral care instruments, together with the associated advantageous features and/or beneficial effects described herein, could likewise be achieved. Other oral care instruments, for example, include those used in dental drilling, polishing, and grinding; oral suction instruments, oral surgical instruments; and other instruments used in the oral cavity which are powered by motorized devices and especially electrical devices.

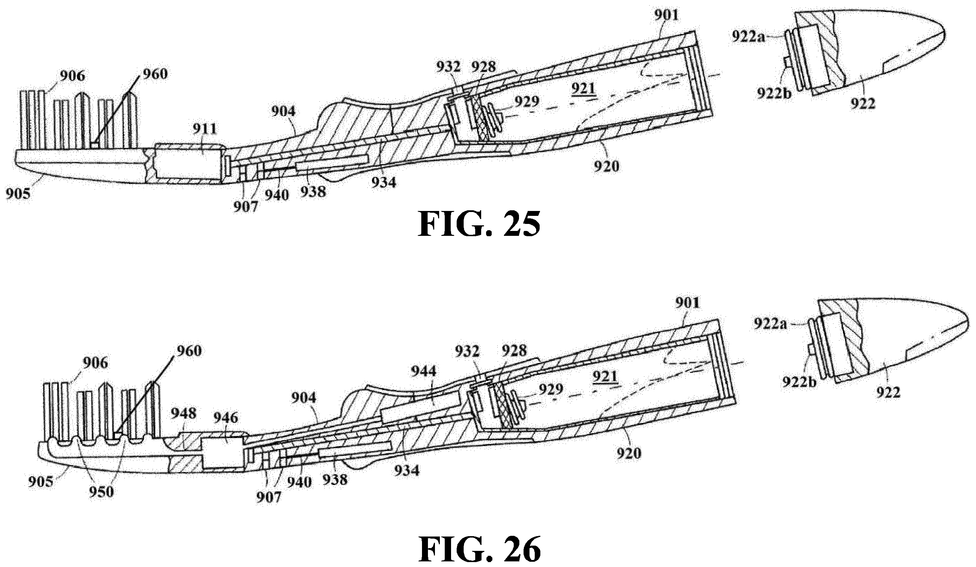

The representative toothbrush illustrated in FIG. 25 has a handle 901 and a head 905 carrying one or more cleaning elements, which are depicted in FIG. 25 as a plurality of bristles 906. Also illustrated is a neck 904 located between, and connecting, handle 901 and head 905. The bristles 906, as shown, form clusters that are anchored to the head 905 and provide a profiled brushing surface with their free ends. Other bristle configurations are of course possible, as well as removable/exchangeable bristle clusters. Different types of cleaning elements (e.g., elastomeric wipers, nodules, pointed structures, etc.) may also be carried on head 905 instead of, or in addition to, bristles.

The neck 904 is provided with electrical conducting elements 907 (e.g., an anode and a cathode) that are exposed to the exterior surface of the toothbrush. In other embodiments, the electrical conducting elements 907 can be located on the head 905, for example on the surface opposite that which carries bristles 906. The use of electrical conducting elements 907 on different parts of the toothbrush is also possible. A plurality of electrical conducting elements 907 can also be incorporated in various positions to activate the instrument in the event that sufficient electrical conductivity is established between any given pair(s) of electrical conducting elements 907 located at any desired position. Integrated in the region of the neck 904 which is adjacent to the head 905 is a LED driver 911 that is operably connected to LED 960. The motorized device 911 is operably connected, via electrical connections 934 in the neck 904 to a power source (e.g., a battery, not shown), which may be accommodated in the handle 901. Operably connected and operably connectable refer to the ability of the electrical connections, or other elements, to readily form an electrical circuit (e.g., when a switch is depressed or when a power source is connected or installed). Operably connected and operably connectable may also refer to the ability of mechanical components to be connected to one another in such a manner as to allow or provide for physical movement of one or more elements. The LED driver may be alternatively incorporated in the head 905 or handle 901 of the toothbrush. In representative embodiments, electrical connections 934 may be metal wire or electrically conductive plastic tracks.

In particular embodiments where the toothbrush uses a vibratory device, it will have a vibratory element which can be in the form of an eccentric, which produces mechanical vibrations and can be rotated about an axis located in the longitudinal direction of the toothbrush. Alternatively, instead of an eccentric which can be driven in rotation, it would also be possible to have a vibratory element which can be driven in a translational manner. Otherwise, the bristle-carrying head 905 can be arranged such that it can be moved in relation to the neck 904 in order for the latter, in the case of vibrations produced by motorized device 911, to move in relation to the rest of the toothbrush.

As shown, also accommodated in the handle 901 is a sheath or sleeve 920 which extends in the longitudinal direction of the handle 901 and is made of electrically conductive material. In the representative embodiment shown, both the handle 901 and the sleeve 920 are open to the rear, thus forming a cavity 921 which can be closed from the rear by a closure part 922 and into which it is possible to insert a battery, such as a commercially available, non-rechargeable cylindrical battery, with a defined voltage (e.g. 1.5 V), as the power or voltage source for LED driver 911. It would also be possible, however, for a button cell or for a rechargeable storage batter to be used as the power source. An external power source such as a conventional electrical outlet or a combination of voltage sources may be employed as the power source.

Also shown in the particular illustrative embodiment of FIG. 25 is a spring contact 929 for a positive pole of a battery (not shown), which is fitted in the sleeve 920, on a transverse wall 92S, and is electrically connected to the LED driver 911 through the electrical connections 934 and switch 932, which is installed in the sleeve 920 and can be actuated from the outside of the handle 901. Switch 932 may also be, for example, a magnetic switch pulse switch or a pulse switch arranged on a printed circuit board with further electronic components that store the switching state. In other embodiments, closure part 922 can itself act as a switch, such that electrical contact between the power source and LED driver 911 is established or interrupted by turning closure part 922 to alter the position of contact surface 922b relative to the negative pole of a battery.

It is to be appreciated, as discussed in greater detail below, that switch 932 is not necessary due to the ability of the toothbrush to turn on automatically when in the user's mouth. In some embodiments, therefore, the toothbrush can be "switchless" or "buttonless."

Switch 932 may be depressed or adjusted by the user to effect a number of operating modes. For example, in "on" and "off" positions or settings, electrical communication or an electrical circuit between the power source and LED driver 911 may be continually established or continually interrupted, respectively. In the former case, for example, the electrical conducting elements 907 may be bypassed to allow continuous operation of motorized device 911, regardless of the presence of a conductive medium between electrical conducting elements 907. Switch 932 may also have a position corresponding to conditional completion of the electrical circuit.

Also as shown in FIG. 25, the closure part 922 is provided with a threaded stub 922a made of an electrically conductive material, which may be the same material (e.g., a metal such as copper or a conductive plastic) used for the electrical conducting elements 907, electrical connections 934, spring contact 929, and/or sleeve 920. Closure part 922 can be screwed into the handle 901 and/or into the sleeve 920 by way of said threaded stub 922a. The threaded stub 922a is provided with a contact surface 922b which, with the closure part 922 screwed in, comes into abutment against the negative pole of a battery (not shown) when inserted into the sleeve 920. During operation of the motorized toothbrush, this negative pole is electrically connected to LED driver 911 via the threaded stub 922a, the sleeve 920 itself, and electrical connections 934 connecting sleeve 920 to LED driver 911. It would also be possible, instead of through the use of sleeve 920, for the power from the negative pole to be transmitted in some other way, for example using wires or electrically conductive plastic tracks. Instead of the rear closure part 922 being screwed to the handle 901, it would, of course, also be possible to have some other type of releasable connection (e.g. plug-in connection, bayonet connection, etc.) and a corresponding configuration of the contact part interacting with the negative pole of the battery.

One representative characteristic of the oral environment which differs significantly from the surrounding or ambient "non-use" environment is electrical conductivity, which increases directionally with the concentration of electrolytes in the surrounding medium (e.g., saliva). In some embodiments, this "non-use" environment may even include rinsing or submersing the portion of the instrument that is normally placed in the mouth (e.g., the head 905 of the toothbrush) in water (e.g., for pre-wetting or rinsing purposes), since the electrical conductivity of saliva is higher than that of water. This difference can thus be utilized to allow the instrument to "detect" when it is being used and thereby operate in an automatic mode.

Additionally, the combination of water, saliva, and dentifrice (e.g., toothpaste or other ingredient that is generated in the mouth during use of the instrument) often affords even a significantly higher electrical conductivity than saliva alone. This is due to the generation of ions, often in large concentrations, from typical oral care products, including tooth fluoridating, whitening, and/or remineralization products which contain or form aqueous cations, such as sodium (Na⁺), potassium (K⁺), calcium (Ca⁺²), magnesium (Mg⁺²), iron (Fe⁺³), etc. and anions, such as phosphate (PO₄⁻³), diphosphate (P₂O₇⁻⁴), carbonate (CO₃⁻²), fluoride (F⁻), chloride (Cl⁻), etc.

In view of the above, the increase in electrical conductivity surrounding a portion of the toothbrush, e.g., head 905 or head 905 and neck 904, when placed in the mouth, can be used to complete an electrical circuit, together with an electrical power or voltage source such as an external electrical outlet or an internal battery to activate LED driver 911, causing LED 960 to turn on.

In an "auto" position or setting, LED driver 911 is powered by the power source only in the event that sufficient electrical conductivity (e.g., a threshold level of conductivity, or sufficiently low resistance) exists between electrical conducting elements 907 in the neck 904. The required electrical conductivity, as needed for the "conditional completion" of the electrical circuit to power motorized device it, may be provided, for example, by an electrolyte solution containing ions (e.g., calcium, phosphate, fluoride, or peroxide ions) such as that generated from a combination of saliva, water, and toothpaste existing in the oral environment during use. When the electrical conductivity between conducting elements 907 is no longer present, the electrical circuit is incomplete, thereby deactivating LED driver 911 and LED 960. Thus, in an "auto" or automatic operating mode, LED driver 911 and LED 960 will not be activated when the toothbrush is stored since air is the medium between electrical conducting elements 907. According to some embodiments, when the brush is being rinsed outside the mouth, the water between electrical conducting elements 907 will not have sufficient electrical conductivity to activate LED driver 911 and LED 960.

According to some embodiments, it may be desired to require that the electrolyte solution (e.g., saliva or a water/saliva/toothpaste mixture), to which the toothbrush is exposed during use, have a threshold (or minimum) level of conductivity before LED driver 911 is activated. This threshold level of conductivity, for example, may be based on a threshold (or minimum) current needed to activate LED driver 911. This threshold conductivity, required to automatically turn on the toothbrush, may be associated with the electrical conductivity of saliva alone or an electrolyte solution having a relatively higher conductivity (e.g., an aqueous solution of toothpaste) or lower (e.g., a mixture of saliva and water) conductivity. For example, the threshold conductivity may be associated with a standard or model electrolyte solution designed to mimic the electrical conductivity of saliva having one or more specified, additional concentrations of dissolved ions such as calcium phosphate, fluoride, peroxide, and other ions or mixtures of ions.

In this manner, the automatic functioning of the oral care instrument can be made more or less sensitive to the particular conditions or conditions associated with the environment in which the instrument is used (i.e., the "use" condition(s) required to activate the instrument). It is also possible that the sensitivity of the instrument can be adjusted by, set by, or tailored to, the user (e.g., to avoid either activation of the instrument during "non-use" conditions or non-activation during "use" conditions) and thereby ensure effective functioning of the instrument in automatic mode.

In certain embodiments, the change in conductivity of the medium between electrical conducting elements 907 is measured by a sensing device 938, such as a circuit board 938 or other suitable sensing device, connected to electrical conducting elements 907 by electrical connections 940. In certain embodiments, sensing device 938 may measure the drop in resistance between conducting elements 907. When the conductivity change reaches a preset value as detected by sensing device 938, switch 932 may be activated so as to complete the electrical circuit to power LED driver 911. In such an embodiment, the electrical circuit need not include the electrolyte solution between conducting elements 907. That is, the electrolyte solution is used as a trigger to activate switch 932 by way of sensing device 938, but does not actually form part of the electrical circuit that powers LED driver 911.

In other embodiments, switch 932 could be activated based on the differential change in conductivity between conducting elements 907. It is to be appreciated that the level of electrolyte in the medium will vary from person to person, and/or may vary based on the formula of the oral care solution used. In such embodiments, these variations will not affect the current level delivered to the motor. Thus, for example, when using a sensitivity type toothpaste product having 5% KNO₃, the toothbrush would not operate differently than when used with a standard toothpaste product having a lower ionic strength.

According to other embodiments, when exposed to a solution with a threshold level of electrical conductivity, LED driver 911 and, therefore, the LED 960 itself may be set or adjusted (e.g., using a timer) to activate for a minimum duration. This ensures that the toothbrush or other instrument will function for at least enough time to effectively accomplish a given task (e.g., tooth cleaning and/or whitening). This also promotes continuous operation, even if contact between the instrument and the electrolyte solution is temporarily lost, for example, when a toothbrush is temporarily removed from the mouth during brushing. The minimum duration for activation of the LED (e.g., two minutes) may be fixed or may otherwise be set or adjusted according to a user's preferences.

As discussed above, the ability of a dental instrument to "activate" (e.g., to turn on a motor) when exposed to the environment in which it is used (e.g., an electrolyte solution in the mouth) can obviate the need for an "on/off" switch or button, creating a simplified operation. Another embodiment of a motorized device activated when conducting elements 907 are exposed to an electrolyte solution is shown in FIG. 26. A reservoir 944 is provided in handle 901 for storing an active agent. Conducting elements 907 are used to activate a pump 946, which causes a predetermined quantity of the active agent to be delivered from reservoir 944 through a channel 948 leading to a plurality of outlets 950 located in head 905. An exemplary delivery system for an active agent is described in US Application Ser. No. 11/457,086, the entire disclosure of which is incorporated herein. Other examples of oral care instruments that can activate an LED upon exposure of conducting elements 907 to an electrolyte solution will become readily apparent to those skilled in the art, given the benefit of this disclosure.

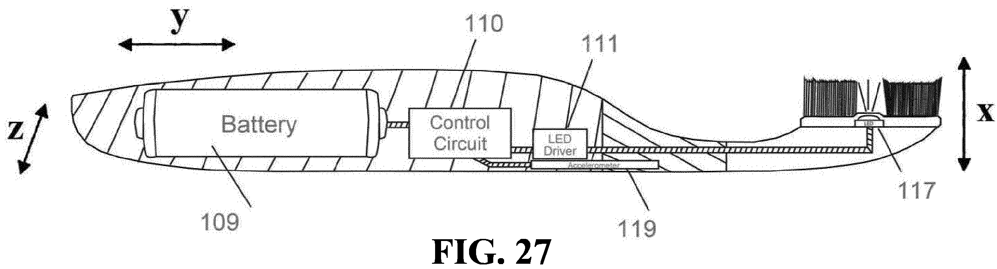

In yet another embodiment, the sensor may be one or more accelerometers within a dental hygiene implement that detect the user's brushing movement and compare it to previously stored brushing profiles to determine if the brush is in use or not. In the embodiment shown in Figure 27, the dental hygiene implement is a toothbrush 100 with an accelerometer 119 connected to a control circuit 110. If the accelerometer 119 detects that the brush is in use, the control circuit 110 will signal to the LED driver 111 to maintain the light source 117 until the brushing time has expired. The brushing time is adjustable, and may be set by either the manufacturer or the user. If the brushing time has expired, then the light source 117 will turn off and an indicator (not shown) will signal to the user that brushing has been completed. In one embodiment, the indicator is an additional LED light located on the handle of the toothbrush. In another embodiment, the indicator is an audio signal relayed to the user.

One or more accelerometers may be installed in the toothbrush to measure the x-, y- and z-axis of the toothbrush at any given moment while the user is brushing his teeth. The control circuit may signal to the LED driver to turn on the light source when the accelerometers indicate that the toothbrush is in an orientation within a predetermined range. In another embodiment of the invention, there is a time delay between the moment the accelerometers indicate that the toothbrush is in a brushing orientation and the moment where the control circuit signals to the LED driver to turn on the light source.

In yet another embodiment, the user may pre-program his brushing pattern to the control circuit such that the LED driver will only activate the light source only when the control circuit recognizes the user's brushing pattern. In one embodiment, the control circuit signals to the LED driver to activate the light source for a predetermined amount of time set by either the manufacturer or user. Alternatively, in another embodiment, the control circuit may continuously signal to the LED driver to keep the light source on until the accelerometer indicates that the user is no longer brushing.

In other embodiments of the invention, the sensor is a combination of two or more of these sensor types.

Embodiments may further include a mechanism for alerting a user that LED 117 is about to be powered on. The, toothbrush 100 may include a light emitter to signal the status of LED 117. The light emitter may be controlled in such a matter as to alert the user that the light is about to come on to full radiant power, through a gradual increase in power from 0% of full power, up to 100% of full power, for a period of between 0.5 seconds to 2 seconds, using a linear, exponential, geometric, logarithmic or other non-linear radiant power curve before a complete and full power is achieved on the light emitting toothbrush. In one embodiment, the light emitter may be an additional LED on toothbrush 100. In various embodiments, the light emitter may operate by providing a short burst of low frequency light pulses or flashes, using a rectangular, saw-tooth, pulsed, sinusoidal or other types of common periodic waveforms, in the range of 2-10 Hertz at partial or full power, for a period of between about 0.5 seconds to about 5 seconds before full power is achieved on the LED in the toothbrush 100. In combination or in the alternative, light emitter may also emit color-coded signals dependent on the amount of power supplied to LED 117. The color of the light emitter may gradually change from a first color to at least a second color as power to the LED 117 gradually increases. Alternatively, the color of the light emitter may change in discrete increments from a first color to at least a second color as LED 117 gradually increases in power. According to the invention the warning light and the therapeutic light are the same. The advantage of a 0.5 -5 second ramp-up of the therapeutic light is that the bright light will trigger the human eye's natural aversion/blink reflex before it poses a hazard to the user. Thus, such a feature acts as a secondary, redundant safety feature in conjunction with the above disclosed eye protection sensors. The light source radiant power may be controlled by adjusting the analog current or through use of pulse width modulation (PWM).

Other mechanisms may be used to alert a user of the power status of LED 117. In one embodiment, a vibrator such as a motor may be incorporated into toothbrush 100. The vibrator may operate by providing a series of pulsed vibration for a short duration of time, such as between about 0.5 seconds to about 2 seconds, before LED 117 is fully powered. In another embodiment, the vibrator may provide a constant vibration with increasing intensity as the power of LED 117 gradually increases. The vibrator may be a motor used to drive the brush head of a motorized toothbrush. In yet another embodiment, toothbrush 100 may further include an auditory device for emitting a sound to alert a user of the power status of LED 117. For example, the speaker may provide a pulsating beep for a short duration to indicate that LED 117 is about to emit at full power.

One or more mechanisms may be included with toothbrush 100 to indicate the upcoming power status of LED 117. These and other identifiers may be useful as a safety feature to prevent a user from inadvertently looking directly at LED 117 while it is fully powered.

It may be possible to inadvertently and momentarily break the interlock safety circuit during normal brushing while the brush head is still in the mouth. For example, when using the current loop embodiment of Figures 1-8, such a momentary break may happen when the brush head is moved from one side of the mouth to the other, breaking the biological circuit between the brush head and the brush handle. This break could cause the light to momentarily shut-off while still in use, creating an unintentional flicker. To prevent this situation, the micro-controller circuit can be programmed to introduce a shut-off delay such that the circuit does not shut-off until the interlock circuit has been broken for more than a predefined period of time. In one embodiment, the predefined period of time is between about 0.1 to about 0.5 seconds. In another embodiment, the break could cause the light to dim for a short period, such as between about 0.1 to about 0.5 seconds before actually shutting off. If the interlock circuit is reengaged during this dimmed time, the light goes back to full power without the need to alert the user.

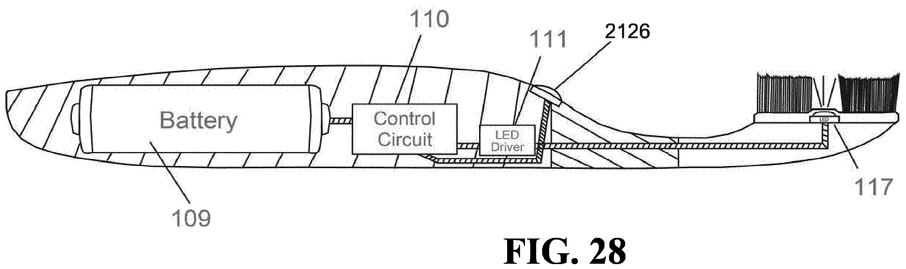

These embodiments and others may further include a fingerprint module mounted on the handle to verify if the user is authorized. In particular, to keep children from playing with the device and defeating the safety measures that prevent the LED from coming on unless it is in a user's mouth, the fingerprint module may be included to only allow the LED to come on if a finger of a hand of an authorized adult is detected. The fingerprint reader may be a button or simply a surface. Various fingerprint sensors are known and can be used in combination with the embodiments disclosed. The fingerprint reader preferably includes a memory for storage of fingerprint data of authorized users and sufficient processing capability to compare stored fingerprint data with new data read as a user attempts to use the toothbrush. Alternatively, the toothbrush could include a data link as in the "internet of things" to compare the fingerprint data remotely and return a "match" or "no match" decision. In the embodiment shown in Figure 28, the dental hygiene implement is a toothbrush 100 having a LED 117 at the brush head connected to a LED driver 111 located in the handle of the toothbrush. The control circuit 110 is connected to the LED driver 111 as well as a battery 109 and a fingerprint module 2126. The fingerprint module 2126 may be initialized to recognize a user, with such information stored in the control circuit 110. Subsequent uses of the toothbrush will require the user to verify his identity by placing his fingerprint on the fingerprint module 2126.

In one embodiment, if the user's identity is correct, then the user may proceed to insert the brush head into his mouth and the LED will turn on in response to the installed sensor (not shown). If the user's identity is incorrect, then the LED will not turn on even if the installed sensor detects pressure, moisture, capacitance, etc. In another embodiment, the toothbrush 100 may further include an on/off switch where the fingerprint module and LED remain completely inactive in the "off" setting. In yet another embodiment, the toothbrush 100 further includes an additional indicator to indicate whether the user is authorized. This indicator may be a visual light source that turns green when a user is authorized to use the brush. A red light source may be activated to indicate that the user is not authorized to use the toothbrush. Other types of indicators may be used, such as an audio indicator.

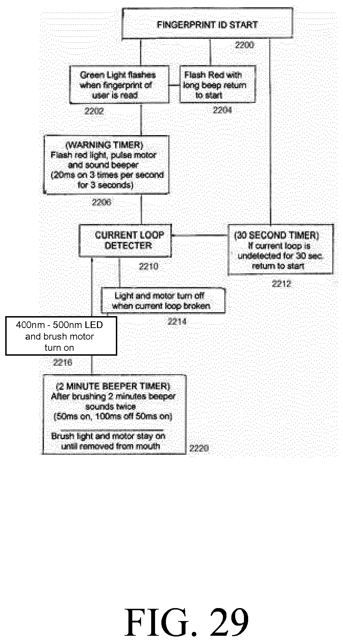

Figure 29 illustrates one example of a toothbrush having a fingerprint module for authorization. The fingerprint module may be presented as a start button for the user to depress and hold for a short duration 2200. The duration may be between about 1 to about 3 seconds. An indicator may be provided to alert the user that the fingerprint has been read. In the example provided, a green light flashes once the fingerprint is read 2202. If an error occurs, the light may flash red and emit a sound 2204. Once the fingerprint is authorized, the light emitter may flash red and provide a pulsing vibration and sound to alert the user that the LED is about to become fully powered 2206. Simultaneously, the current loop detector determines whether the toothbrush is being inserted into the mouth (or a detector using any of the mechanisms as described in the embodiments above) 2210. If no current is detected after a preset time interval, then the toothbrush shuts off 2212. If a current is detected, then the LED and brush motor are activated 2216. A timer indicates when a sufficient duration of brushing has been reached by beeping an auditory signal to the user 2220. Once the user removes the brush from the mouth, the current loop is broken 2214. If the toothbrush remains out of mouth for 30 seconds, the system turns off and the fingerprint reader will need to be used to reactivate system 2212.

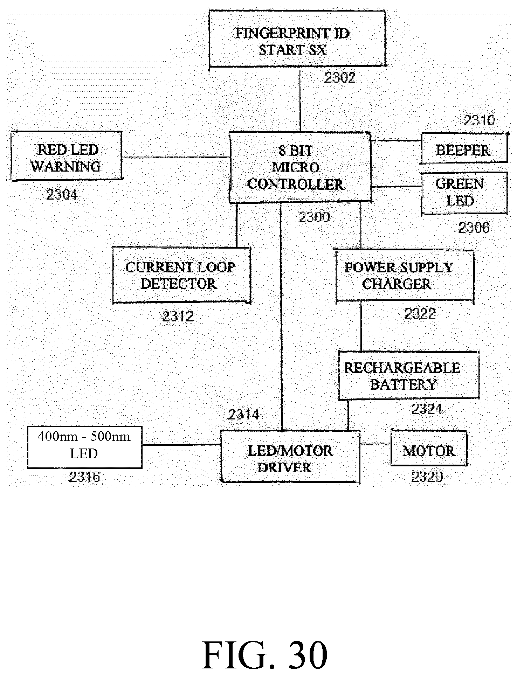

Figure 30 illustrates one embodiment of how the fingerprint module and status indicators are connected with other components of the toothbrush using a circuit block diagram. The microcontroller 2300 is wired to the following components: the fingerprint module 2302, a red LED 2304, a green LED 2306, a beeper 2310, a current loop detector 2312, a LED/motor driver 2314, and a power supply 2322/2324. The LED/motor driver 2314 is responsible for activating the UV LED 2316 and the brush head motor 2320. LED/motor driver 2314 is also connected to the power supply 2322/2324.

Of course, the fingerprint module and warning feature may be used with non-motorized toothbrushes.

In another embodiment, the fingerprint module 2126 acts as the sensor for toothbrush 100. The LED will turn on once an authorized user places his fingerprint over the module. In this embodiment, it is intended for the user to place the fingerprint over the module only after the brush head is inserted within the mouth.

The primary purpose of an interlock sensor is to protect the user's eyes from sudden and unexpected bright light. However, conventional designs allow only for either an on or off state, which presents two problems. First, the sudden transition from an "off" state to an "on" state could happen inadvertently, such as when the user applies pressure to the brush head with one hand whilst holding the handle in the other hand. This could expose the user's eyes to an unexpected bright burst of light. Second, the sudden transition from an "on" state to an "off" state could cause flickering during normal operation, such as when the brush head is moved from one side of the mouth to the other side of the mouth. This could shut off the light even when the brush is still in the user mouth. Furthermore, if a ramp-up sequence is implemented, this sequence could be triggered at the wrong time when the brush head is still inside the user's mouth.

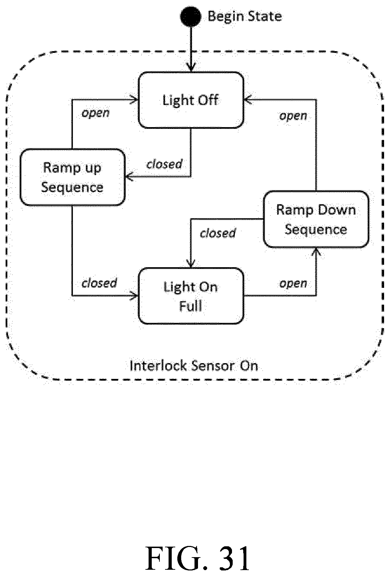

These inadvertent problems could occur regardless of the types of sensor used. To address these problems, the microcontroller (for example Renesas RL78/G12 manufactured by Renesas Electronics) which regulates the functions of the light emitter can be programmed to implement four operating states with respect to light activation according to the state transition diagram depicted in Figure 31.

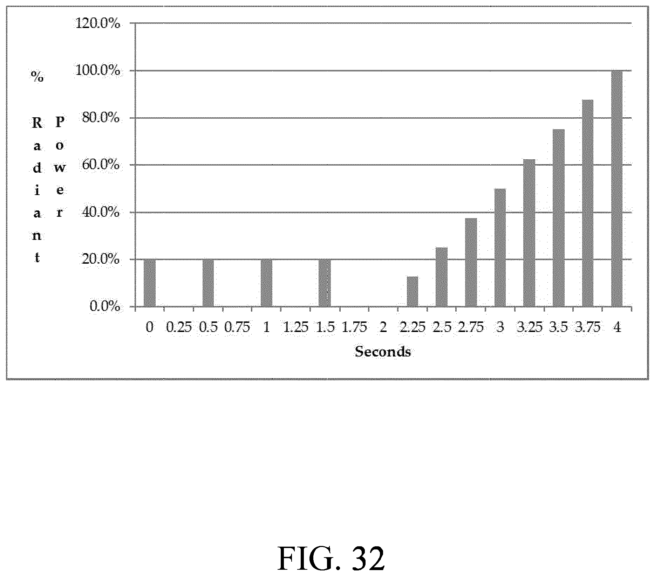

The state transitions correspond to events detected by any of the various sensors which can be used to detect an "in-the-mouth" condition. Figure 31 provides one example of the events that can trigger these transitions. The beginning state for the toothbrush is "light off." If a "closed" event is detected, the state transitions to the Ramp-up sequence. In one embodiment, the ramp up state can only occur immediately after the "light off" state and the interlock sensor detects a closed "in mouth" condition. For example, in one embodiment, the brush head LED emits between 1 and 10 perceptible light pulses of between 0.1 to 0.5 seconds each at between 10% and 50% radiant power output with interludes of between 0.1 to 0.5 seconds between each pulse. Then the power would gradually and continuously increase from no power to full power for a further 0.5 to 5 seconds as depicted in the example Figure 32. Other types on ramp-up sequences can be programmed into the microcontroller, and this example is not limiting. The important characteristics of a ramp up sequence include a gradual increase in radiant power to allow ample time for the user to respond or trigger the eyes natural aversion reflex, such as blinking, if the light source is too bright for the eyes. While the flashing option can be used, the ramp-up without flashing is preferred.

Returning to Figure 31, if the circuit opens during the ramp up sequence the interlock state reverts to light off. If it remains closed at the end of the ramp up sequence the interlock state transitions to "light on full." The light on full state occurs after the successful completion of a ramp-up sequence, or the unsuccessful exit of a ramp-down sequence and the sensor circuit is in a closed condition. Radiant power output is at or near its optimum operating threshold. This state can exit through a circuit open event, in which case the state transitions to the ramp-down sequence. The purpose of the ramp down sequence is to prevent flickering during normal brushing. The ramp down sequence can only occur after the "light on full" state. When the interlock circuit is opened, the brush head light switches to between 10% to 50% radiant output power for a transition period of between 0.1 to 1 second. If the circuit is closed at any time during the ramp down sequence, the state immediately reverts back to the "light on full" state. If the ramp down transition period completes without a "closed" event and the interlock circuit is still open then the interlock state transitions back to "light off."

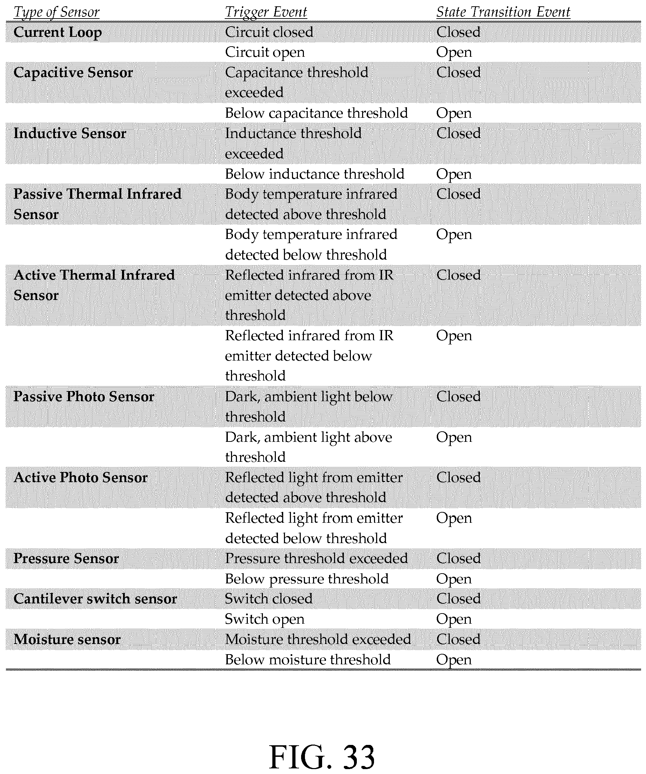

The above software relating to the ramp-up/ramp-down sequence can operate with any type of interlock sensor that can detect an "in-the-mouth" condition to control the light source of a light emitting toothbrush. Such a toothbrush will have at least a portion of the handle and/or at least a portion of the distal end being equipped with a sensor to determine whenever the distal end is outside the environment of the mouth and permits activation of the source of light only when the source of light is inside the mouth of the user, the sensor selected from the group consisting of: a current signal loop which detects current flowing from the brush handle through the body of the user to the brush head, a capacitive sensor which detects current flowing through the body of a user when the brush head or bristles are in contact with the mouth and the handle is in contact with the hand; a capacitive displacement sensor that senses change of position of any conductive target such as the human body; an inductive sensor that uses an inductance loop to measure the proximity of conductors such as the human body; a passive thermal infrared that detects the warmth of the human mouth; a photoelectric sensor that detects reflected IR light emitted and absorbed by the sensor itself; a light sensor that is triggered by darkness inside the mouth; a light sensor that detects the reflection of light from a second light source on the brush when the brush is activated; an ultrasonic and active sonar sensors that uses echo location to detect the confines of the mouth; a magnetic detector that detects the proximity of metals such as the hemoglobin present in blood; a pressure sensor under the brush head that detects movement and pressure of the brush head being pressed against the teeth; a pressure sensor in the neck that detects torque and tension in the neck of the brush due to brushing action; a cantilever switch sensor under the brush head that detects movement and pressure of the brush head being pressed against the teeth; a cantilever switch sensor in the neck that detects torque and tension in the neck of the brush due to brushing action; a moisture sensor to detect a highly moist environment such as the mouth; and a combination of two or more of these sensor types. These sensors and their corresponding events which would alter the state of the safety interlock are depicted in Figure 33.

In another embodiment, the toothbrush employing the current signal loop interlock for safe application of a visible therapeutic light source may be modified to further operate as an ionic toothbrush in combination with the therapeutic properties of blue and/or violet light. Experiments have shown that visible therapeutic light primarily affects Black Pigmented Bacteria (BPB) which are predominantly periodontal pathogenic strains such as; Fusobacterium Nucleatum, Fusobacterium Periodonticum, Porphyromonas Gingivalis, Prevotella Intermedia, Prevotella Melaninogenica, and Prevotella Nigrescens. However, visible therapeutic light has little or no effect on non-pigmented bacteria or those strains with a low concentration of pigment. Many pathogenic bacteria fall into this category; for example, Streptococcus Mutans, Streptococcus Sobrinus and Lactobacillus species are common and significant contributors to tooth decay (dental caries). Visible therapeutic light is ineffective against these and other non-pigmented strains.

The combination of visible therapeutic light and ionic action provides two non-mechanical modalities that disrupt pathogenic bacteria in places where a traditional toothbrush can't reach, such as between the teeth or below the gum line. This combination therapy enables all of the pathogenic strains to be targeted including periodontal pathogens as wells as pathogens that cause dental caries (tooth decay). Moreover, while visible therapeutic light can attrite and weaken the biofilm by killing Fusobacterium Nucleatum, a key plaque aggregant, an ionic toothbrush can also disaggregate bacteria using electrostatic forces. Therefore, visible therapeutic light and ionic action work in combination to disrupt the cohesion of plaque biofilm providing better cleaning results than either technology applied individually or separately.

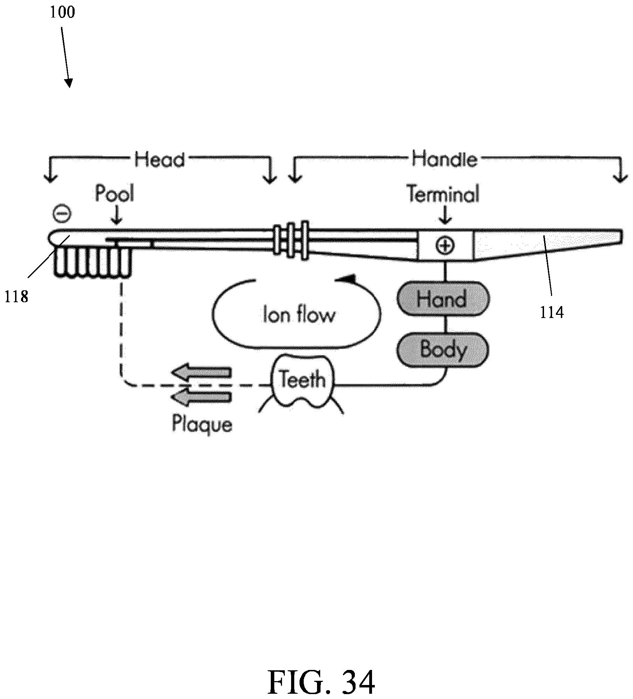

Ionic therapy using the signal loop equipped toothbrush may be implemented by providing the negatively charged anode in the brush head and the positively charged cathode in the brush handle, as seen in Figure 34. Using a DC current with the signal loop sensor, the potential difference between the anode and cathode may be between about 1 volt and about 10 volts. In one embodiment, the potential difference is about 3 volts. Higher therapeutic currents may be achieved by ensuring the signal loop has low internal resistance compared to the resistance of the human body.

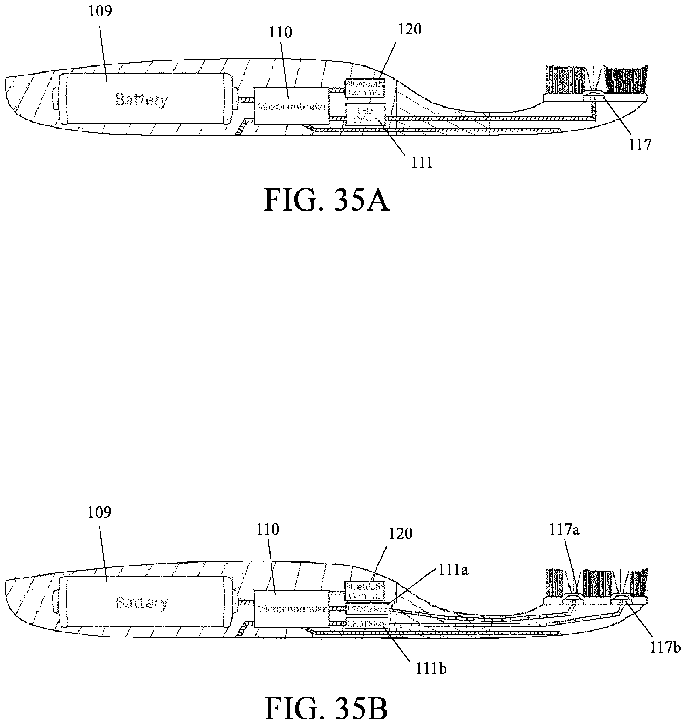

As seen in Figure 35A, embodiments of toothbrush 100 may include a communication system 120 for sending and receiving signals to another device. For instance, communication system 120 may be a near-field communication module or Bluetooth^{®} communication module used for pairing with a computing device. One suitable example of a Bluetooth^{®} communication module compatible with toothbrush 100 is Bluetooth^{®} V4.1 Smart (Low Energy) Single Mode Module sold by TDK as SESUB-PAN-D14580. Nonlimiting examples of computing devices that toothbrush 100 may pair with include desktops, laptops, tablets or smartphones.

A software application may be installed on one or more computing devices to interact with toothbrush 100. The software may operate in the toothbrush, the paired device, or a further remote computer, or the software operation distributed among more than one of them. Data sent from toothbrush 100 may include its battery power levels, the current default settings, the microcontroller software version, and details of the user's brushing and light therapy events, such as start and end times and radiant power output levels. The data can be captured through the current signal loop or other eye protection sensor and recorded in a memory bank for later use.. The toothbrush 100 may include a 24-hour clock for monitoring brushing and light therapy events. One example of a microcontroller with a clock suitable for inclusion in toothbrush 100 is Renasys RL78/12. The clock can be calibrated using the software application.

Using the software application, the computing device may also send data to toothbrush 100. For example, the software application may enable a user to select various preference settings and modify various features of the toothbrush, including modifying the power levels for the light source and motor, modifying the intensity of ionic potential, defining a fingerprint for an authorized user to be verified with a fingerprint sensor, modifying the ramp-up/ramp-down sequence, selecting a particular light and ionic therapy to be applied while brushing, or updating the onboard programming of the toothbrush. The selected preferences can then be uploaded to toothbrush 100 from the computing device. The computing device may also provide an identification key once it is paired with toothbrush 100 to ensure that neither is incorrectly paired with another toothbrush or computing device. For embodiments of toothbrush 100 incorporating a fingerprint security feature, the software application may provide security matching of the fingerprint ID to unlock the toothbrush.

The software application may also be used to monitor the brushing habits and progress of a prescribed therapy. In some embodiments, the software application may begin monitoring brushing habits and therapy progress once the toothbrush 100 detects that the brush head 118 is in the mouth using a current loop sensor or other interlock sensor described above. For instance, a user may be prescribed a specific light or ionic therapy by a dentist or physician. The software application is typically programmed to monitor the user's brushing habits and therapy progress each time the user inserts the brush head into his/her mouth.

The software application may include features that provide information regarding the user's brushing and therapy habits. Examples of information provided include the duration of light therapy sessions, radiant power output, the number of light therapy sessions per day, the duration of brushing sessions, the number of brushes per day, the wear state of the brush head (i.e., the percentage of maximum use), comparisons between the user's brush use and the recommendations of the American Dental Association (ADA) or other recommender, comparisons of brush use to consumer averages, and when to replace the brush head (e.g., after a predefined number of minutes of use or number of brushings). The data can be presented in raw form or as averages or other statistical measures.

The data may also be shared with others, such as a friend, parent, dentist or overseeing physician. Data shared by a patient to a dentist or physician may include progress of a prescribed light and ionic therapy. In one embodiment, the data may be sent via an email or text message. In another embodiment, shared data may be received from a software application. Users may also share their data via social media. Still, other means of application data sharing can be used in alternative embodiments. An overseeing dentist can highlight gum inflammation or gum pockets affecting certain teeth at the front side or back of the tooth using the dentist's universal teeth numbering system chart (see figure 42) and feed that data directly into the smart phone application or a cloud based application that is synchronized with the smart phone application. The dentist can then prescribe a recommended daily amount of light therapy in specific locations of the mouth using this application so that the patient can self-monitor their progress or share the therapeutic data with their physician through a data synchronized application. Software can be configured to monitor toothbrush position and inclination using sensors to monitor position and orientation such as accelerometers, IR temperature sensors, as taught in patents (e.g. WO 2002083257, US20120246858, WO 2014202250) so that the duration and intensity of light therapy applied to each of the afflicted gum pocket areas can be calculated from sensor data and compared to dentist prescribed therapy.

By sharing data with the software application, the overseeing dentist or physician can adjust and monitor the application of a prescribed therapy. For instance, the number of volts of potential difference can be adjusted by the therapist for a prescribed ionic therapy. The number of minutes of therapy as well as the number of Joules and watts of current can be monitored over time and adjusted as necessary.

The software application may also alert the user and overseeing health practitioner when a prescribed therapy is not being followed. For instance, the health prescriber could select the minimum number of Joules of light energy per day or a number of minutes at prescribed strength and wavelength per day and the application would alert if this benchmark is not met.

The software application may also provide incentives for users to establish proper brushing or light therapy habits. In one embodiment, the software application may include a system for rewarding users. For instance, the system may comprise an in-application currency that may be redeemed for rewards based on brushing habits. By way of example, a user's parent may give permission for the user to play a video game or watch a favorite TV show if the user accumulates a certain number of points that are earned over a number of brushing periods.

In some scenarios, the toothbrush and computing device may not be in range with each other and therefore cannot exchange data. Embodiments of the toothbrush can store collected data into the memory of microcontroller 110 until it is within range of a paired computing device to exchange data. When the toothbrush and computing device are in range during use, data may be actively exchanged while brushing.

The toothbrush and software application may further connect with and other accessories using communication system 120. For instance, the software application may monitor data collected from accessory sensor systems such as other oral biometric sensor devices. Examples include detecting: volatile sulfur compounds (VSC) in a person's breath which are associated with bad breath and halitosis; the presence of an active gum infection or other oral infection; changes in the oral flora with respect to caries progression focused on detection of bacterial pathogens, measurements of salivary and dental plaque pH, salivary buffering capacity and flow rate, among other parameters; monitoring the dynamics of periodontal disease, such as differentiating between progressive versus arrested states, responses to treatment, or monitoring of the presence of specific bacteria; detecting and monitoring of premalignant lesions; detecting viruses in the oral mucosa; and monitoring tooth enamel demineralization/remineralization. A prescribed therapy may be added or modified based on the results gathered from accessory sensor systems.

In another embodiment of the software application, the whiteness of the user's teeth can be measured and calculated algorithmically compared to a standard teeth whitening chart using color-adjusted photographs (for example, taken through a smart phone app). When the photograph also contains color calibration cards that can be recognized by the software through distinctive iconography, the accuracy of the teeth shade calculations is significantly improved. This may also be monitored and reported through the software application and may be shared with an overseeing therapist, such as a cosmetic dentist.

Figure 35B shows an embodiment wherein toothbrush 100 may include a first light source 117a and a second light source 117b. First light source 117a may comprise a blue and/or violet light having a wavelength in the range of between about 400 nm and about 500 nm, "isible therapeutic light." Second light source 117b may comprise a red light having a wavelength in the range of between about 600 nm and about 1000 nm, referred to herein as "infrared therapeutic light." Each light source is connected to its respective LED driver 111, which is connected to microcontroller 110.

During brushing, first light source 117a and second light source 117b may emit light simultaneously. In another embodiment, toothbrush 100 may include a single light source 117 incorporating two or more wavelengths or between about 400 nm to about 500 nm and about 600 nm to about 1000 nm, i.e. both visible therapeutic light and infrared therapeutic light. Toothbrush 100 may further include a Bluetooth^{®} communications module 120 for modifying the light intensity, wavelength and other settings as described above.

Figure 36 illustrates another embodiment wherein the light source is installed on the handle of toothbrush 100. In the embodiment shown, light source 190 is installed in the handle 114. Brush head 118 is a separate piece having a light pipe 196 that aligns with source 190 to carry light through an aperture 198 adjacent to bristles 113. Brush head 118 may be detachable from the handle 114 using a bayonet mount. Other mounting connections can be used such as male/female press fits, with either the handle or the neck of the distal end having the female fitting and the other having the male fitting.

Light pipe 196 may be housed in a non-conductive plastic sheath. A light source concentrator 192 is installed with light source 190 that increases the intensity of the emitted light 194 from light source 190. Preferably, both the light source concentrator 192 and light pipe 196 should be less than about 8 mm in diameter to remain comfortable for the user to use.

In some embodiments, the light source concentrator 192 comprises a parabolic concentrator that uses total internal reflection to direct emitted light 194 along light pipe 196 to aperture 198. In operation, the parabolic concentrator takes the form of a shifted and tilted parabola of revolution in accordance with the edge ray principle. One example of a suitable shape for a light source concentrator would be Part Number 10356 from Carclo Optics, with its size modified to be installed onto the handle 114 of toothbrush 100.

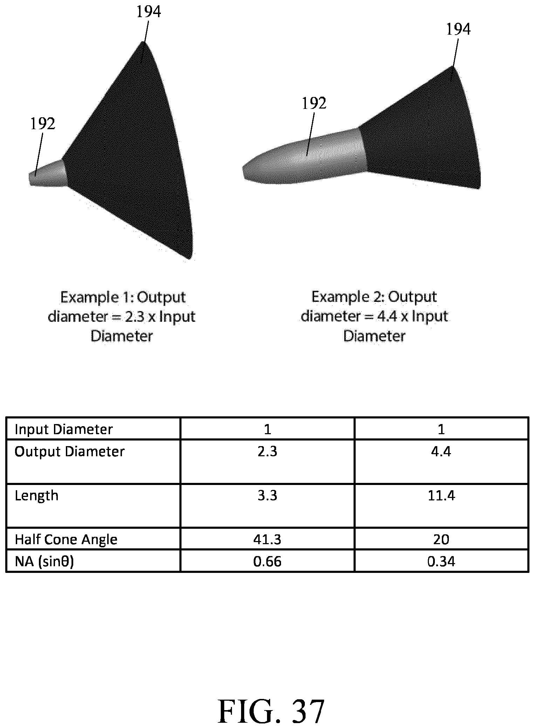

Preferably, the total internal reflection of the surface should have an efficiency of greater than about 80% to minimize transmission losses. Transmission losses may be minimized by keeping the angle of incidence (i.e., the angle at which the emitted light 194 enters light pipe 196) within a range where total internal reflection is achieved. This may be accomplished by selecting the proper sizing for the light source concentrator. In one embodiment, the toothbrush may include a 1 mm LED with a light source concentrator 192 and a light pipe 196 having a shared diameter of 4.4 mm. As seen in Figure 37, the angle of incidence is a function of the ratio between the input diameter and output diameter and length of the light source concentrator.

Other mechanisms may be used to transmit emitted light 194 through light pipe 196. For example, light pipe 196 may comprise a mirrored or other curved surface to facilitate guiding emitted light 194 to aperture 198. Other beam-steering components, such as lenses, diffractive optics and prisms, may be installed in the brush head 118 to further control how emitted light 194 is distributed through the light pipe 196 and aperture 198.

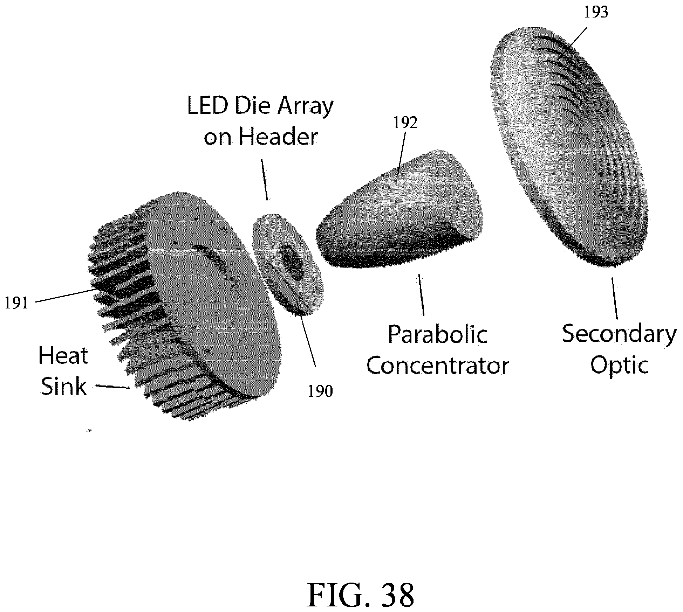

Figure 38 shows an enlarged, exploded view of various components for the light assembly in Figure 36. The light assembly includes light source 190 attached to a heat sink 191 to dissipate heat and a light source concentrator attached to the other end of light source 190. In the embodiment shown, light source 190 comprises an array of LEDs on a PCB board and light source concentrator 192 comprises a parabolic concentrator. A secondary optic 193 may be used to further steer emitted light. In other embodiments, light source 190 may comprise a laser diode instead of a light-emitting diode. For example, light source 190 may be a vertical-cavity surface-emitting laser (VCSEL). The VCSEL may be used as a potential source for emitting red and/or infrared light, "infrared therapeutic light." If an unfocussed laser diode is used, the light source concentrator will likely not be required due to the inherently narrower beam concentration of laser diodes.

Figure 39 shows an embodiment of toothbrush 100 wherein light pipe 196 is encased in electrically conductive plastic 195 (e.g., a carbon-based plastic). Light source 190 is connected to LED driver 111, and LED driver 111 is connected to microcontroller 110 for regulating the functions of the light source. Microcontroller 110 is connected to the battery 109, the conductive plastic backing plate 197 on handle 114, and to the conductive plastic 195 forming the brush head 118 to form an electrical circuit when the user holds the handle 114 and the brush head 118 is placed in the mouth of the user. This condition is detected through the current signal loop to enable the therapeutic light.

An enlarged and cross-sectional view of another embodiment of brush head 118 is shown in Figure 39. This brush head and the brush head shown in Figure 40 are used with a handle like the handle shown in Figure 38. Light pipe 196 is encased in conductive plastic 133 and surrounded by a non-conductive plastic layer 134 forming brush head 118. Conductive plastic 133 is exposed at the distal end of brush head 118 at aperture 198. As the user grabs the handle 114 and inserts the brush head 118 into the mouth, an electrical circuit is completed by the connection of the mouth in contact with plastic 133 that is also connected to the circuit in the handle (not shown), and the light source 190 is activated.

Another embodiment of brush head 118 is shown in Figure 41, wherein the microcontroller 110 of Figure 39 is connected to an embedded metal wire 116 that is exposed at the distal end of brush head 118 and on the surface of the handle 114. Light pipe 196 is encased in non-conductive plastic 112. An electrical circuit is formed when the user grabs the handle 114 and inserts bristles 113 into the mouth, wherein metal wire 116 comes into contact with the user's mouth and hand to establish the electrical circuit to the microcontroller 110.

Installing the light source 190 in the handle 114 provides several benefits. For instance, the light source 190 may be driven at greater power levels when installed at the handle 114 since there is no source of heat in the user's mouth. It also enables smaller and more compact designs, and allows the manufacturer to further modify the light distribution at aperture 198 using various secondary optics 193. The sizing of aperture 198 as well as the light intensity of emitted light 194 may also be modified; for example, the aperture may be sized larger than a standard LED diode. Having a larger aperture enables the emitted light to cover a greater surface area of the user's mouth while brushing. In one embodiment, aperture 198 covers between about 10% to about 50% of the surface area of brush head 118.

Certain modifications and improvements will occur to those skilled in the art upon reading the foregoing description. It should be understood that all such modifications and improvements have been omitted for the sake of conciseness and readability, but are properly within the scope of the following claims.

## Claims

1. A toothbrush (100), comprising
a battery (186),
a source (117, 190) of visible therapeutic light with a wavelength in the range of 400nm to 500nm,
electronics (110, 111) coupled to the battery and to the source of visible therapeutic light to supply electricity from the battery to the source of visible therapeutic light at current and voltage that causes the source of visible therapeutic light to emit light, the toothbrush including a handle (114) and a distal end, the distal end including bristles (132) on the distal end;
wherein the source (117) of visible therapeutic light is located at the distal end, or wherein the source (190) of visible therapeutic light is installed in the handle (114), an aperture (198) for emitting the light is located at the distal end and a light pipe (196) installed at the distal end guides the light from the source (190) of visible therapeutic light to the aperture (198),
the electronics including a control circuit (110) and a sensor or combination of sensors (210, 240, 248, 256, 280, 306, 314, 334) configured to determine whenever the distal end is outside the environment of the mouth and to permit activation of the source (117, 190) of visible therapeutic light only when the sensor or combination of sensors detect that the distal end of the device has been placed in the mouth, and
wherein the control circuit (110) is configured to activate the source (117, 190) of visible therapeutic light using a 0.5 to 5 second long ramp-up sequence that gradually increases the radiant power of the source of visible therapeutic light to allow ample time for the user to respond or trigger the eyes natural aversion reflex, such as blinking, if the light is too bright for the eyes, the source (117, 190) of visible therapeutic light thereby being an alarm that warns the user of the power status of the source of visible therapeutic light before the source of visible therapeutic light reaches full power.

2. A toothbrush (100) as claimed in claim 1 wherein the ramp-up sequence comprises the source of visible therapeutic light emitting a short burst of low frequency light pulses or flashes, using a rectangular, saw-tooth, pulsed, sinusoidal or other types of common periodic waveforms, in the range of 2-10 Hertz at partial or full power, for a period of between 0.5 seconds to 5 seconds before full power is achieved

3. A toothbrush (100) as claimed in claim 1 wherein the ramp-up sequence comprises the source of visible therapeutic light emitting between 1 and 10 perceptible light pulses of between 0.1 to 0.5 seconds each at between 10% and 50% radiant power output with interludes of between 0.1 to 0.5 seconds between each pulse.

4. A toothbrush (100) as claimed in claim 1 wherein the ramp-up sequence incorporates a gradually increasing radiant power using a linear, exponential, geometric, logarithmic or other non-linear radiant power curve before a complete and full power is achieved.

5. A toothbrush (100) as claimed in claim 1 wherein the sensor has a closed state when detecting the distal end is inside the mouth of the user and an open state at other times, and the control circuit (110) has a ramp-down sequence that begins when the sensor switches from the closed state to the open state and returns to the visible therapeutic light on full power when the sensor switches from the open state to the closed state during the ramp-down sequence.

6. A toothbrush (100) as claimed in claim 1 wherein the ramp-up sequence incorporates a short burst of low frequency light pulses or flashes of the visible therapeutic light.

7. A toothbrush (100) as claimed in claim 5 wherein the control circuit supplies power to the source of visible therapeutic light at 10% to 50% radiant output power for a transition period of between 0.1 to 1 seconds during the ramp-down sequence when the closed circuit becomes open.

8. A toothbrush (100) as claimed in claim 1 wherein the alarm further comprises a vibrator to warn the user of the power status of the source of visible therapeutic light.

9. A toothbrush (100) as claimed in claim 5 wherein the control circuit (110) supplies between about 10% to about 50% power to the source of visible therapeutic light for a transition period of between about 0.1 to about 1 second during the ramp-down sequence.

10. A toothbrush (100) as claimed in claim 1 wherein the toothbrush further incorporates an infrared therapeutic light source (117b) with a wavelength in the range of 600nm to 1000nm.

11. A toothbrush (100) as claimed in claim 1 wherein the toothbrush is a manual toothbrush or motorized power toothbrush.

12. A toothbrush (100) as claimed in claim 1 wherein the sensor or combination of sensors is selected from the group consisting of: a current signal loop (115, 116) which detects current flowing from the handle (114) through the body of the user to the distal end; a capacitive sensor (210) which detects current flowing through the body of a user when a brush head comprising the distal end or the bristles are in contact with the mouth and the handle is in contact with the hand; a capacitive displacement sensor (240) that senses change of position of any conductive target such as the human body; an inductive sensor (248) that uses an inductance loop to measure the proximity of conductors such as the human body; one or more accelerometers (119) that detect the user's brushing movement, a passive thermal infrared sensor (256) that detects the warmth of the human mouth; a photoelectric sensor that detects reflected IR light emitted and absorbed by the sensor itself; a light sensor (306) that is triggered by darkness inside the mouth; a light sensor (314) that detects the reflection of light from a second light source (316) on the toothbrush when the toothbrush is activated; an ultrasonic and active sonar sensors (334) that uses echo location to detect the confines of the mouth; a magnetic detector that detects the proximity of metals such as the hemoglobin present in blood; a pressure sensor under a brush head comprising the distal end that detects movement and pressure of the brush head being pressed against the teeth; a pressure sensor in a neck of the toothbrush connecting the handle and a brush head comprising the distal end that detects torque and tension in the neck of the toothbrush due to brushing action; a moisture sensor to detect a highly moist environment such as the mouth; and a combination of two or more of these sensor types.

13. A toothbrush (100) as claimed in claim 1 wherein the source (190) of visible therapeutic light is located in the handle (114) of the toothbrush and directs light through the light pipe (196) of a detachable brush head comprising the distal end of the toothbrush, and wherein the detachable brush head emits the light out of the aperture (198) in the distal end.

## Patentansprüche

1. Zahnbürste (100), umfassend:
eine Batterie bzw. einen Akku (186),
eine Quelle (117, 190) eines sichtbaren therapeutischen Lichts mit einer Wellenlänge im Bereich von 400 nm bis 500 nm,
eine Elektronik (110, 111), die mit der Batterie bzw. dem Akku und der Quelle sichtbaren therapeutischen Lichts gekoppelt ist, um Elektrizität von der Batterie bzw. dem Akku an die Quelle sichtbaren therapeutischen Lichts mit einer Stromstärke und einer Spannung zu liefern, die bewirken, dass die Quelle sichtbaren therapeutischen Lichts Licht emittiert, wobei die Zahnbürste einen Handgriff (114) und ein distales Ende aufweist, wobei das distale Ende Borsten (132) auf dem distalen Ende aufweist,
wobei die Quelle (117) sichtbaren therapeutischen Lichts an dem distalen Ende angeordnet ist, oder wobei die Quelle (190) sichtbaren therapeutischen Lichts in dem Handgriff (114) eingebaut ist, eine Öffnung (198) zum Emittieren des Lichts an dem distalen Ende angeordnet ist, und einen Lichtleiter (196), der an dem distalen Ende eingebaut ist, der Licht von der Quelle (190) sichtbaren therapeutischen Lichts zur Öffnung (198) leitet,
wobei die Elektronik eine Steuerschaltung (110) und einen Sensor oder eine Kombination von Sensoren (210, 240, 248, 256, 280, 306, 314, 334) aufweist, die dazu konfiguriert sind festzustellen, wann das distale Ende außerhalb der Umgebung des Munds ist, und eine Aktivierung der Quelle (117, 190) sichtbaren therapeutischen Lichts nur dann zu gestatten, wenn der Sensor oder die Kombination von Sensoren erfasst, dass das distale Ende der Vorrichtung in dem Mund angeordnet wurde, und
wobei die Steuerschaltung (110) dazu konfiguriert ist, die Quelle (117, 190) sichtbaren therapeutischen Lichts unter der Verwendung einer 0,5 bis 5 Sekunden langen Einschaltsequenz zu aktivieren, die die Strahlungsleistung der Quelle sichtbaren therapeutischen Lichts allmählich ansteigen lässt, um dem Benutzer mehr als genügend Zeit zu geben, auf den natürlichen Abwehrreflex der Augen, wie zum Beispiel Blinzeln, zu reagieren oder diesen auszulösen, wenn das Licht für die Augen zu hell ist, wobei die Quelle (117, 190) sichtbaren therapeutischen Lichts dadurch ein Alarm ist, der den Benutzer hinsichtlich des Leistungsstatus der Quelle sichtbaren therapeutischen Lichts warnt, bevor die Quelle sichtbaren therapeutischen Lichts ihre volle Leistung erreicht.

2. Zahnbürste (100) gemäß Anspruch 1, wobei die Einschaltsequenz umfasst, dass die Quelle sichtbaren therapeutischen Lichts eine kurze Folge niederfrequenter Lichtpulse oder Blitze emittiert, unter der Verwendung rechteckiger, sägezahnförmiger, gepulster, sinusförmiger oder anderer Typen üblicher periodischer Wellen in dem Bereich von 2 bis 10 Hertz mit einer Teilleistung oder vollen Leistung über einen Zeitraum von zwischen 0,5 Sekunden bis 5 Sekunden, bevor die volle Leistung erreicht wird.

3. Zahnbürste (100) gemäß Anspruch 1, wobei die Einschaltsequenz umfasst, dass die Quelle sichtbaren therapeutischen Lichts zwischen 1 und 10 wahrnehmbare Lichtpulse von zwischen 0,1 bis 0,5 Sekunden mit jeweils zwischen 10% und 50% Strahlungsleistung mit Pausen von zwischen 0,1 bis 0,5 Sekunden jeweils zwischen den Pulsen emittiert.

4. Zahnbürste (100) gemäß Anspruch 1, wobei die Einschaltsequenz ein allmähliches Erhöhen der Strahlungsleistung unter der Verwendung einer linearen, exponentiellen, geometrischen, logarithmischen oder anderen nicht-linearen Strahlungsleistungskurve beinhaltet, bevor eine vollständige und volle Leistung erreicht wird.

5. Zahnbürste (100) gemäß Anspruch 1, wobei der Sensor einen geschlossenen Zustand hat, wenn erfasst wird, dass sich das distale Ende im Inneren des Mundes des Benutzers befindet, und einen offenen Zustand zu anderen Zeiten hat, und die Steuerschaltung (110) eine Abschaltsequenz hat, die beginnt, wenn der Sensor von dem geschlossenen Zustand in den offenen Zustand schaltet, und zu dem sichtbaren therapeutischen Licht mit voller Leistung zurückkehrt, wenn der Sensor während der Abschaltsequenz von dem offenen Zustand in den geschlossenen Zustand schaltet.

6. Zahnbürste (100) gemäß Anspruch 1, wobei die Einschaltsequenz eine kurze Folge niederfrequenter Lichtpulse oder Blitze des sichtbaren therapeutischen Lichts beinhaltet.

7. Zahnbürste (100) gemäß Anspruch 5, wobei die Steuerschaltung Leistung an die Quelle sichtbaren therapeutischen Lichts mit 10% bis 50% Strahlungsleistung über einen Übergangszeitraum von zwischen 0,1 bis 1 Sekunden während der Abschaltsequenz liefert, wenn die geschlossene Schaltung offen wird.

8. Zahnbürste (100) gemäß Anspruch 1, wobei der Alarm ferner einen Vibrator beinhaltet, um den Benutzer über den Leistungsstatus der Quelle sichtbaren therapeutischen Lichts zu warnen.

9. Zahnbürste (100) gemäß Anspruch 5, wobei die Steuerschaltung (110) zwischen ungefähr 10% bis ungefähr 50% Leistung an die Quelle sichtbaren therapeutischen Lichts über einen Übergangszeitraum von zwischen ungefähr 0,1 bis ungefähr 1 Sekunde während der Abschaltsequenz liefert.

10. Zahnbürste (100) gemäß Anspruch 1, wobei die Zahnbürste ferner eine Quelle (117b) therapeutischen Infrarotlichts mit einer Wellenlänge im Bereich von 600 nm bis 1000 nm enthält.

11. Zahnbürste (100) gemäß Anspruch 1, wobei die Zahnbürste eine manuelle Zahnbürste oder eine motorisierte elektrische Zahnbürste ist.

12. Zahnbürste (100) gemäß Anspruch 1, wobei der Sensor oder die Kombination von Sensoren aus der Gruppe ausgewählt ist, die aus Folgendem besteht: eine Strom-Signalschleife (115, 116), die einen Strom erfasst, der von dem Handgriff (114) durch den Körper des Benutzers zum distalen Ende fließt; ein kapazitiver Sensor (210), der einen Strom erfasst, der durch den Körper des Benutzers fließt, wenn ein Bürstenkopf, der das distale Ende aufweist, oder die Borsten mit der Hand in Kontakt sind; ein kapazitiver Wegsensor (240), der eine Änderung der Position eines beliebigen leitfähigen Ziels, wie zum Beispiel des menschlichen Körpers, erfasst; ein induktiver Sensor (248), der eine Induktivität dazu verwendet, die Nähe von Leitern, wie zum Beispiel des menschlichen Körpers zu erfassen; einer oder mehrere Beschleunigungssensoren (119), die die Bürstbewegung des Benutzers erfassen; ein pyroelektrischer Sensor (256), der die Wärme des menschlichen Mundes erfasst; ein photoelektrischer Sensor, der reflektiertes IR-Licht erfasst, das von dem Sensor selbst emittiert und von diesem erfasst wird; ein Lichtsensor (306), der durch Dunkelheit im Inneren des Mundes ausgelöst wird; ein Lichtsensor (314), der die Reflexion von Licht von einer zweiten Lichtquelle (316) auf der Zahnbürste erfasst, wenn die Zahnbürste aktiviert wird; ein Ultraschall- und aktiver Sonar-Sensor (334), der eine Echolotung dazu verwendet, die Grenzen des Mundes zu erfassen; ein Magnetdetektor, der die Nähe von Metallen, wie zum Beispiel in dem Blut vorhandenes Hämoglobin, erfasst; ein Drucksensor unter einem Bürstenkopf, der das distale Ende aufweist, der eine Bewegung und einen Druck des Bürstenkopfs erfasst, der gegen die Zähne gedrückt wird; ein Drucksensor in einem Hals der Zahnbürste, der den Handgriff mit einem Bürstenkopf verbindet, der das distale Ende aufweist, der ein Drehmoment und eine Spannung in dem Hals der Zahnbürste aufgrund eines Bürstvorgangs erfasst; ein Feuchtesensor zum Erfassen einer höchst feuchten Umgebung, wie zum Beispiel des Munds; und eine Kombination aus zwei oder mehr dieser Sensortypen.

13. Zahnbürste (100) gemäß Anspruch 1, wobei die Quelle (190) sichtbaren therapeutischen Lichts in dem Handgriff (114) der Zahnbürste angeordnet ist und Licht durch den Lichtleiter (196) eines abnehmbaren Bürstenkopfs richtet, der das distale Ende der Zahnbürste aufweist, und wobei der abnehmbare Bürstenkopf das Licht aus der Öffnung (198) in dem distalen Ende emittiert.

## Revendications

1. Brosse à dents (100), comprenant
une pile (186),
une source (117, 190) de lumière thérapeutique visible avec une longueur d'onde dans la plage de 400 nm à 500 nm,
des équipements électroniques (110, 111) couplés à la pile et à la source de lumière thérapeutique visible pour fournir de l'électricité depuis la pile à la source de lumière thérapeutique visible avec un courant et une tension tels qu'ils amènent la source de lumière thérapeutique visible à émettre une lumière, la brosse à dents incluant une poignée (114) et une extrémité distale, l'extrémité distale incluant des poils (132) sur l'extrémité distale ;
dans laquelle la source (117) de lumière thérapeutique visible est située au niveau de l'extrémité distale, ou dans laquelle
la source (190) de lumière thérapeutique visible est installée dans la poignée (114), un orifice (198) pour émettre la lumière est situé au niveau de l'extrémité distale et un conduit de lumière (196) installé au niveau de l'extrémité distale guide la lumière depuis la source (190) de lumière thérapeutique visible vers l'orifice (198),
les équipements électroniques incluant un circuit de commande (110) et un capteur ou une combinaison de capteurs (210, 240, 248, 256, 280, 306, 314, 334) configurés pour déterminer quand l'extrémité distale est à l'extérieur de l'environnement de la bouche et pour autoriser une activation de la source (117, 190) de lumière thérapeutique visible uniquement lorsque le capteur ou la combinaison de capteurs détectent que l'extrémité distale du dispositif a été placée dans la bouche, et
dans laquelle le circuit de commande (110) est configuré pour activer la source (117, 190) de lumière thérapeutique visible à l'aide d'une séquence d'accélération de puissance longue de 0,5 à 5 secondes qui augmente graduellement la puissance rayonnante de la source de lumière thérapeutique visible afin de laisser suffisamment de temps à l'utilisateur pour répondre ou déclencher le réflexe d'aversion naturel de l'œil, tel un clignement, si la lumière est trop intense pour les yeux, la source (117, 190) de lumière thérapeutique visible constituant ainsi une alarme qui alerte l'utilisateur sur l'état de puissance de la source de lumière thérapeutique visible avant que la source de lumière thérapeutique visible n'atteigne une pleine puissance.

2. Brosse à dents (100) selon la revendication 1, dans laquelle la séquence d'accélération de puissance comprend le fait que la source de lumière thérapeutique visible émette une courte rafale d'impulsions ou d'éclairs lumineux à basse fréquence, à l'aide d'une forme d'onde rectangulaire, en dents de scie, pulsée, sinusoïdale ou d'autres types de forme d'onde périodique courante, dans la plage de 2-10 Hertz à puissance partielle ou pleine, pendant une période allant de 0,5 seconde à 5 secondes avant qu'une pleine puissance ne soit atteinte.

3. Brosse à dents (100) selon la revendication 1, dans laquelle la séquence d'accélération de puissance comprend le fait que la source de lumière thérapeutique visible émette entre 1 et 10 impulsions lumineuses perceptibles d'entre 0,1 et 0,5 seconde chacune à une sortie de puissance rayonnante d'entre 10 % et 50 % avec des interludes d'entre 0,1 et 0,5 seconde entre chaque impulsion.

4. Brosse à dents (100) selon la revendication 1, dans laquelle la séquence d'accélération de puissance incorpore une puissance rayonnante augmentant graduellement à l'aide d'une courbe de puissance rayonnante linéaire, exponentielle, géométrique, logarithmique ou d'une autre courbe de puissance rayonnante non linéaire avant qu'une puissance complète et pleine ne soit atteinte.

5. Brosse à dents (100) selon la revendication 1, dans laquelle le capteur présente un état fermé lorsqu'il détecte que l'extrémité distale est à l'intérieur de la bouche de l'utilisateur et un état ouvert à d'autres moments, et le circuit de commande (110) présente une séquence de décélération de puissance qui commence lorsque le capteur commute depuis l'état fermé vers l'état ouvert et retourne vers la lumière thérapeutique visible à pleine puissance lorsque le capteur commute depuis l'état ouvert vers l'état fermé pendant la séquence de décélération de puissance.

6. Brosse à dents (100) selon la revendication 1, dans laquelle la séquence d'accélération de puissance incorpore une courte rafale d'impulsions ou d'éclairs lumineux à basse fréquence de la lumière thérapeutique visible.

7. Brosse à dents (100) selon la revendication 5, dans laquelle le circuit de commande fournit une puissance à la source de lumière thérapeutique visible à une puissance de sortie de puissance rayonnante de 10 % à 50 % pendant une période de transition d'entre 0,1 et 1 seconde pendant la séquence de décélération de puissance lorsque le circuit fermé devient ouvert.

8. Brosse à dents (100) selon la revendication 1, dans laquelle l'alarme comprend en outre un vibrateur pour alerter l'utilisateur sur l'état de puissance de la source de lumière thérapeutique visible.

9. Brosse à dents (100) selon la revendication 5, dans laquelle le circuit de commande (110) fournit une puissance d'entre environ 10 % et environ 50 % à la source de lumière thérapeutique visible pendant une période de transition d'entre environ 0,1 et environ 1 seconde pendant la séquence de décélération de puissance.

10. Brosse à dents (100) selon la revendication 1, dans laquelle la brosse à dents incorpore en outre une source de lumière thérapeutique infrarouge (117b) avec une longueur d'onde dans la plage de 600 nm à 1 000 nm.

11. Brosse à dents (100) selon la revendication 1, dans laquelle la brosse à dents est une brosse à dents manuelle ou une brosse à dents à puissance motorisée.

12. Brosse à dents (100) selon la revendication 1, dans laquelle le capteur ou la combinaison de capteurs est sélectionné(e) dans un groupe consistant en : une boucle de signal de courant (115, 116) qui détecte un courant circulant depuis la poignée (114) à travers le corps de l'utilisateur vers l'extrémité distale ; un capteur capacitif (210) qui détecte un courant circulant à travers le corps d'un utilisateur lorsqu'une tête de brosse comprenant l'extrémité distale ou les poils sont en contact avec la bouche et la poignée est en contact avec la main ; un capteur de déplacement capacitif (240) qui capte un changement de position de toute cible conductrice tel le corps humain ; un capteur inductif (248) qui utilise une boucle d'inductance pour mesurer la proximité de conducteurs tel le corps humain ; un ou plusieurs accéléromètres (119) qui détectent un mouvement de brossage de l'utilisateur ; un capteur infrarouge thermique passif (256) qui détecte la chaleur de la bouche humaine ; un capteur photoélectrique qui détecte une lumière IR réfléchie émise et absorbée par le capteur lui-même ; un capteur de lumière (306) qui est déclenché par l'obscurité à l'intérieur de la bouche ; un capteur de lumière (314) qui détecte la réflexion de lumière depuis une seconde source de lumière (316) sur la brosse à dents lorsque la brosse à dents est activée ; un capteur ultrasonique et sonar actif (334) qui utilise l'écholocalisation pour détecter les confins de la bouche ; un détecteur magnétique qui détecte la proximité de métaux telle l'hémoglobine présente dans le sang, un capteur de pression sous une tête de brosse comprenant l'extrémité distale qui détecte un mouvement et une pression de la tête de brosse qui est appuyée contre les dents ; un capteur de pression dans un col de la brosse à dents reliant la poignée et une tête de brosse comprenant l'extrémité distale qui détecte un couple et une tension dans le col de la brosse à dents du fait d'une action de brossage ; un capteur d'humidité pour détecter un environnement hautement humide telle la bouche ; et une combinaison de deux ou plusieurs de ces types de capteur.

13. Brosse à dents (100) selon la revendication 1, dans laquelle la source (190) de lumière thérapeutique visible est située dans la poignée (114) de la brosse à dents et dirige la lumière à travers le conduit de lumière (196) d'une tête de brosse amovible comprenant l'extrémité distale de la brosse à dents, et dans laquelle la tête de brosse amovible émet la lumière hors de l'orifice (198) dans l'extrémité distale.
